# EUROPEAN PATENT SPECIFICATION

(11) **EP 3 077 406 B1**
(45) Date of publication and mention of the grant of the patent: **10.07.2019**
(21) Application number: 14821372.1
(22) Date of filing: 05.12.2014
(51) Int. Cl.: C07H 21/04

(54) **COMPOSITIONS AND METHODS FOR CAPPING RNA**
ZUSAMMENSETZUNGEN UND VERFAHREN ZUM VERSCHLIESSEN VON RNA
COMPOSITIONS ET MÉTHODES DE COIFFAGE D'ARN

(30) Priority: 05.12.2013 US 201361912367 P; 23.12.2013 US 201361920380 P; 23.05.2014 US 201462002564 P; 13.06.2014 US 201462011918 P
(43) Date of publication of application: 12.10.2016
(73) Proprietor: New England Biolabs, Inc., Ipswich, MA 01938 (US)
(72) Inventor: SCHILDKRAUT, Ira, Boxford, MA 01921 (US); ETTWILLER, Laurence, Beverly, MA 01915 (US); BUSWELL, John, Byfield, MA 01922 (US); YIGIT, Erbay, Boxford, MA 01921 (US)
(74) Representative: Griffin, Philippa Jane
(86) International application number: PCT/US2014/068737
(87) International publication number: WO 2015/085142

(56) References cited:
- WO-A2-2007/120863
- TYZOON K NOMANBHOY ET AL: "Active site of an aminoacyl-tRNA synthetase dissected by energy-transfer-dependent fluorescence", BIOORGANIC & MEDICINAL CHEMISTRY LETTERS, vol. 11, no. 12, 1 June 2001 (2001-06-01), pages 1485-1491, XP055179341, ISSN: 0960-894X, DOI: 10.1016/S0960-894X(01)00127-5
- ERIK ANDERSON ET AL: "A fluorescent GTP analog as a specific, high-precision label of microtubules", BIOTECHNIQUES, vol. 51, no. 1, 1 July 2011 (2011-07-01), XP055092401, ISSN: 0736-6205, DOI: 10.2144/000113703
- DATABASE MEDLINE [Online] US NATIONAL LIBRARY OF MEDICINE (NLM), BETHESDA, MD, US; 15 July 1990 (1990-07-15), SHUMAN S: "Catalytic activity of vaccinia mRNA capping enzyme subunits coexpressed in Escherichia coli.", Database accession no. NLM2164022 & SHUMAN S: "Catalytic activity of vaccinia mRNA capping enzyme subunits coexpressed in Escherichia coli.", THE JOURNAL OF BIOLOGICAL CHEMISTRY 15 JUL 1990, vol. 265, no. 20, 15 July 1990 (1990-07-15), pages 11960-11966, ISSN: 0021-9258

### SEQUENCE LISTING

<110> New England Biolabs, Inc.
<120> COMPOSITIONS AND METHODS FOR CAPPING RNA
<130> NEB-374-PCT
<150> 61/912,367
   <151> 2013-12-05
<150> 61/920,380
   <151> 2013-12-23
<150> 62/002,564
   <151> 2014-05-23
<150> 62/001,918
   <151> 2014-06-13
<160> 13
<170> PatentIn version 3.5
<210> 1
   <211> 844
   <212> PRT
   <213> Artificial Sequence
<220>
   <223> Synthetic construct
<400> 1
   Met Asp Ala Asn Val Val Ser Ser Ser Thr Ile Ala Thr Tyr Ile Asp
   1               5                   10                  15
   Ala Leu Ala Lys Asn Ala Ser Glu Leu Glu Gln Arg Ser Thr Ala Tyr
               20                  25                  30
   Glu Ile Asn Asn Glu Leu Glu Leu Val Phe Ile Lys Pro Pro Leu Ile
           35                  40                  45
   Thr Leu Thr Asn Val Val Asn Ile Ser Thr Ile Gln Glu Ser Phe Ile
       50                  55                  60
   Arg Phe Thr Val Thr Asn Lys Glu Gly Val Lys Ile Arg Thr Lys Ile
   65                  70                  75                  80
   Pro Leu Ser Lys Val His Gly Leu Asp Val Lys Asn Val Gln Leu Val
                   85                  90                  95
   Asp Ala Ile Asp Asn Ile Val Trp Glu Lys Lys Ser Leu Val Thr Glu
               100                 105                 110
   Asn Arg Leu His Lys Glu Cys Leu Leu Arg Leu Ser Thr Glu Glu Arg
   115 120 125
   His Ile Phe Leu Asp Tyr Lys Lys Tyr Gly Ser Ser Ile Arg Leu Glu
   130 135 140
   Leu Val Asn Leu Ile Gln Ala Lys Thr Lys Asn Phe Thr Ile Asp Phe
   145 150 155 160
   Lys Leu Lys Tyr Phe Leu Gly Ser Gly Ala Gln Ser Lys Ser Ser Leu
   165 170 175
   Leu His Ala Ile Asn His Pro Lys Ser Arg Pro Asn Thr Ser Leu Glu
   180 185 190
   Ile Glu Phe Thr Pro Arg Asp Asn Glu Thr Val Pro Tyr Asp Glu Leu
   195 200 205
   Ile Lys Glu Leu Thr Thr Leu Ser Arg His Ile Phe Met Ala Ser Pro
   210 215 220
   Glu Asn Val Ile Leu Ser Pro Pro Ile Asn Ala Pro Ile Lys Thr Phe
   225 230 235 240
   Met Leu Pro Lys Gln Asp Ile Val Gly Leu Asp Leu Glu Asn Leu Tyr
   245 250 255
   Ala Val Thr Lys Thr Asp Gly Ile Pro Ile Thr Ile Arg Val Thr Ser
   260 265 270
   Asn Gly Leu Tyr Cys Tyr Phe Thr His Leu Gly Tyr Ile Ile Arg Tyr
   275 280 285
   Pro Val Lys Arg Ile Ile Asp Ser Glu Val Val Val Phe Gly Glu Ala
   290 295 300
   Val Lys Asp Lys Asn Trp Thr Val Tyr Leu Ile Lys Leu Ile Glu Pro
   305 310 315 320
   Val Asn Ala Ile Asn Asp Arg Leu Glu Glu Ser Lys Tyr Val Glu Ser
   325 330 335
   Lys Leu Val Asp Ile Cys Asp Arg Ile Val Phe Lys Ser Lys Lys Tyr
   340 345 350
   Glu Gly Pro Phe Thr Thr Thr Ser Glu Val Val Asp Met Leu Ser Thr
   355 360 365
   Tyr Leu Pro Lys Gln Pro Glu Gly Val Ile Leu Phe Tyr Ser Lys Gly
       370                 375                 380
   Pro Lys Ser Asn Ile Asp Phe Lys Ile Lys Lys Glu Asn Thr Ile Asp
   385                 390                 395                 400
   Gln Thr Ala Asn Val Val Phe Arg Tyr Met Ser Ser Glu Pro Ile Ile
                   405                 410                 415
   Phe Gly Glu Ser Ser Ile Phe Val Glu Tyr Lys Lys Phe Ser Asn Asp
               420                 425                 430
   Lys Gly Phe Pro Lys Glu Tyr Gly Ser Gly Lys Ile Val Leu Tyr Asn
           435                 440                 445
   Gly Val Asn Tyr Leu Asn Asn Ile Tyr Cys Leu Glu Tyr Ile Asn Thr
       450                 455                 460
   His Asn Glu Val Gly Ile Lys Ser Val Val Val Pro Ile Lys Phe Ile
   465                 470                 475                 480
   Ala Glu Phe Leu Val Asn Gly Glu Ile Leu Lys Pro Arg Ile Asp Lys
                   485                 490                 495
   Thr Met Lys Tyr Ile Asn Ser Glu Asp Tyr Tyr Gly Asn Gln His Asn
               500                 505                 510
   Ile Ile Val Glu His Leu Arg Asp Gln Ser Ile Lys Ile Gly Asp Ile
           515                 520                 525
   Phe Asn Glu Asp Lys Leu Ser Asp Val Gly His Gln Tyr Ala Asn Asn
       530                 535                 540
   Asp Lys Phe Arg Leu Asn Pro Glu Val Ser Tyr Phe Thr Asn Lys Arg
   545                 550                 555                 560
   Thr Arg Gly Pro Leu Gly Ile Leu Ser Asn Tyr Val Lys Thr Leu Leu
                   565                 570                 575
   Ile Ser Met Tyr Cys Ser Lys Thr Phe Leu Asp Asp Ser Asn Lys Arg
               580                 585                 590
   Lys Val Leu Ala Ile Asp Phe Gly Asn Gly Ala Asp Leu Glu Lys Tyr
           595                 600                 605
   Phe Tyr Gly Glu Ile Ala Leu Leu Val Ala Thr Asp Pro Asp Ala Asp
       610                 615                 620
   Ala Ile Ala Arg Gly Asn Glu Arg Tyr Asn Lys Leu Asn Ser Gly Ile
   625                 630                 635                 640
   Lys Thr Lys Tyr Tyr Lys Phe Asp Tyr Ile Gln Glu Thr Ile Arg Ser
                   645                 650                 655
   Asp Thr Phe Val Ser Ser Val Arg Glu Val Phe Tyr Phe Gly Lys Phe
               660                 665                 670
   Asn Ile Ile Asp Trp Gln Phe Ala Ile His Tyr Ser Phe His Pro Arg
           675                 680                 685
   His Tyr Ala Thr Val Met Asn Asn Leu Ser Glu Leu Thr Ala Ser Gly
       690                 695                 700
   Gly Lys Val Leu Ile Thr Thr Met Asp Gly Asp Lys Leu Ser Lys Leu
   705                 710                 715                 720
   Thr Asp Lys Lys Thr Phe Ile Ile His Lys Asn Leu Pro Ser Ser Glu
                   725                 730                 735
   Asn Tyr Met Ser Val Glu Lys Ile Ala Asp Asp Arg Ile Val Val Tyr
               740                 745                 750
   Asn Pro Ser Thr Met Ser Thr Pro Met Thr Glu Tyr Ile Ile Lys Lys
           755                 760                 765
   Asn Asp Ile Val Arg Val Phe Asn Glu Tyr Gly Phe Val Leu Val Asp
       770                 775                 780
   Asn Val Asp Phe Ala Thr Ile Ile Glu Arg Ser Lys Lys Phe Ile Asn
   785                 790                 795                 800
   Gly Ala Ser Thr Met Glu Asp Arg Pro Ser Thr Arg Asn Phe Phe Glu
                   805                 810                 815
   Leu Asn Arg Gly Ala Ile Lys Cys Glu Gly Leu Asp Val Glu Asp Leu
               820                 825                 830
   Leu Ser Tyr Tyr Val Val Tyr Val Phe Ser Lys Arg
           835                 840
<210> 2
   <211> 287
   <212> PRT
   <213> Artificial Sequence
<220>
   <223> Synthetic construct
<400> 2
   Met Asp Glu Ile Val Lys Asn Ile Arg Glu Gly Thr His Val Leu Leu
   1               5                   10                  15
   Pro Phe Tyr Glu Thr Leu Pro Glu Leu Asn Leu Ser Leu Gly Lys Ser
               20                  25                  30
   Pro Leu Pro Ser Leu Glu Tyr Gly Ala Asn Tyr Phe Leu Gln Ile Ser
           35                  40                  45
   Arg Val Asn Asp Leu Asn Arg Met Pro Thr Asp Met Leu Lys Leu Phe
       50                  55                  60
   Thr His Asp Ile Met Leu Pro Glu Ser Asp Leu Asp Lys Val Tyr Glu
   65                  70                  75                  80
   Ile Leu Lys Ile Asn Ser Val Lys Tyr Tyr Gly Arg Ser Thr Lys Ala
                   85                  90                  95
   Asp Ala Val Val Ala Asp Leu Ser Ala Arg Asn Lys Leu Phe Lys Arg
               100                 105                 110
   Glu Arg Asp Ala Ile Lys Ser Asn Asn His Leu Thr Glu Asn Asn Leu
           115                 120                 125
   Tyr Ile Ser Asp Tyr Lys Met Leu Thr Phe Asp Val Phe Arg Pro Leu
       130                 135                 140
   Phe Asp Phe Val Asn Glu Lys Tyr Cys Ile Ile Lys Leu Pro Thr Leu
   145                 150                 155                 160
   Phe Gly Arg Gly Val Ile Asp Thr Met Arg Ile Tyr Cys Ser Leu Phe
                   165                 170                 175
   Lys Asn Val Arg Leu Leu Lys Cys Val Ser Asp Ser Trp Leu Lys Asp
               180                 185                 190
   Ser Ala Ile Met Val Ala Ser Asp Val Cys Lys Lys Asn Leu Asp Leu
           195                 200                 205
   Phe Met Ser His Val Lys Ser Val Thr Lys Ser Ser Ser Trp Lys Asp
       210                 215                 220
   Val Asn Ser Val Gln Phe Ser Ile Leu Asn Asn Pro Val Asp Thr Glu
   225                 230                 235                 240
   Phe Ile Asn Lys Phe Leu Glu Phe Ser Asn Arg Val Tyr Glu Ala Leu
                   245                 250                 255
   Tyr Tyr Val His Ser Leu Leu Tyr Ser Ser Met Thr Ser Asp Ser Lys
               260                 265                 270
   Ser Ile Glu Asn Lys His Gln Arg Arg Leu Val Lys Leu Leu Leu
           275                 280                 285
<210> 3
   <211> 58
   <212> PRT
   <213> Artificial Sequence
<220>
   <223> Synthetic construct
<400> 3
<210> 4
   <211> 26
   <212> PRT
   <213> Artificial Sequence
<220>
   <223> Synthetic construct
<400> 4
   Lys Arg Arg Trp Lys Lys Asn Phe Ile Ala Val Ser Ala Ala Asn Arg
   1               5                   10                  15
   Phe Lys Lys Ile Ser Ser Ser Gly Ala Leu
               20                  25
<210> 5
   <211> 6
   <212> PRT
   <213> Artificial Sequence
<220>
   <223> Synthetic construct
<400> 5
   His His His His His His
   1 5
<210> 6
   <211> 8
   <212> PRT
   <213> Artificial Sequence
<220>
   <223> Synthetic construct
<400> 6
   Asp Tyr Lys Asp Asp Asp Asp Lys
   1 5
<210> 7
   <211> 9
   <212> PRT
   <213> Artificial Sequence
<220>
   <223> Synthetic construct
<400> 7
   Tyr Pro Tyr Asp Val Pro Asp Tyr Ala
   1 5
<210> 8
   <211> 10
   <212> PRT
   <213> Artificial Sequence
<220>
   <223> Synthetic construct
<400> 8
   Glu Gln Lys Leu Ile Ser Glu Glu Asp Leu
   1 5 10
<210> 9
   <211> 14
   <212> PRT
   <213> Artificial Sequence
<220>
   <223> Synthetic construct
<400> 9
   Gly Lys Pro Ile Pro Asn Pro Leu Leu Gly Leu Asp Ser Thr
   1               5                   10
<210> 10
   <211> 8
   <212> PRT
   <213> Artificial Sequence
<220>
   <223> Synthetic construct
<400> 10
   Trp Ser His Pro Gln Phe Glu Lys
   1               5
<210> 11
   <211> 38
   <212> PRT
   <213> Artificial Sequence
<220>
   <223> Synthetic construct
<400> 11
   Met Asp Glu Lys Thr Thr Gly Trp Arg Gly Gly His Val Val Glu Gly
   1               5                   10                  15
   Leu Ala Gly Glu Leu Glu Gln Leu Arg Ala Arg Leu Glu His His Pro
               20                  25                  30
   Gln Gly Gln Arg Glu Pro
           35
<210> 12
   <211> 15
   <212> PRT
   <213> Artificial Sequence
<220>
   <223> Synthetic construct
<400> 12
   Lys Glu Thr Ala Ala Ala Lys Phe Glu Arg Gln His Met Asp Ser
   1               5                   10                  15
<210> 13
   <211> 51
   <212> PRT
   <213> Artificial Sequence
<220>
   <223> Synthetic construct
<400> 13
   Thr Asn Pro Gly Val Ser Ala Trp Gln Val Asn Thr Ala Tyr Thr Ala
   1               5                   10                  15
   Gly Gln Leu Val Thr Tyr Asn Gly Lys Thr Tyr Lys Cys Leu Gln Pro
               20                  25                  30
   His Thr Ser Leu Ala Gly Trp Glu Pro Ser Asn Val Pro Ala Leu Trp
           35                  40                  45
   Gln Leu Gln
       50

## Description

Prokaryotic and eukaryotic cells contain multiple types of RNA at least some of which can be characterized by different chemical constituents at their 5' ends. Within all cells, RNA polymerase initiates synthesis of RNA with a 5' terminal nucleotide having a 5' triphosphate. 5' monophosphate nucleotides are then successively added to the 3' end. However, for eukaryotes, RNA destined to become messenger RNA is rapidly capped with a ^{7m}G nucleotide linked to the 5' terminus via a 5' triphosphate linkage. This modification is a result of the capping enzyme. In contrast, the messenger RNA of bacteria and archaea maintain their 5' triphosphate. In all kingdoms of life, ribosomal RNA makes up the vast majority of the cell's RNA but its 5' end is generated by endonucleolytic cleavage to leave a 5' monophosphate terminus. Furthermore when RNA is degraded in the cell by RNases, the 5' ends are either 5' monophosphate or 5' hydroxyl groups.

When analyzing the sequence and quantity of specific RNAs, it is desirable to remove ribosomal RNA and degraded RNA from the complex mixture of RNAs. There are currently a number of procedures for ribosomal RNA depletion, but they all suffer some shortcomings. For example, depletion methods commonly require the hybridization of DNA oligonucleotides complementary to ribosomal RNA and removal of the hybrid molecules. This requires customizing the DNA sequence to match the ribosomal RNA. It also requires *a priori* knowledge of the ribosomal sequence. Another procedure that has been used has been the specific enzymatic degradation of 5' monophosphate RNA that would include ribosomal RNA, however this enzymatic reaction has proved inefficient and leaves substantial rRNA and degraded RNA in the mixture.

WO 2007/120863 describes kits and methods for generating 5' capped RNA.

The invention is as defined in the claims.

In general, a compound represented by Formula (I): 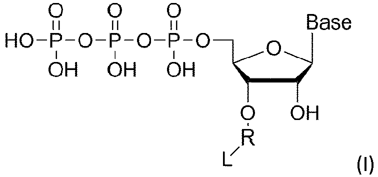 or a salt thereof is provided where the Base is a purine or a pyrimidine, R is a linker; and L is a label. The label is selected from the group consisting of an affinity label, a detection label, a chemoselective group, an oligonucleotide, and a combination thereof. For example, the label can be an affinity label selected from the group consisting of a biotin moiety, desthiobiotin, avidin, streptavidin, protein A, maltose-binding protein, poly-histidine, HA-tag, c-myc tag, FLAG-tag, SNAP-tag, S-tag, and glutathione-S-transferase (GST). In one example of a detection label, the label may be a fluorescent label. In one aspect, the base is a purine where the purine is guanosine, inosine or an analog thereof. In one example, the compound may be 3'-O-(2-aminoethylcarbamoyl) (EDA)-biotin guanosine tri-phosphate (GTP) or 3'-desthiobiotin-tetraethylene glycol (TEG)-GTP.

In one aspect, the invention provides a method for forming a 5' capped labeled RNA as defined in the claims. The method includes combining a preparation comprising uncapped RNA having a 5' diphosphate or 5' triphosphate with a capping enzyme and a labeled modified nucleotide represented by Formula (I): 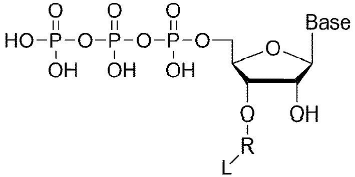 or a salt thereof, wherein: Base is a purine or a pyrimidine, R is a linker, and L is a label selected from the group consisting of an affinity label, a detection label, a chemoselective group, an oligonucleotide, and a combination thereof; so as to convert the uncapped RNA into 5' capped labeled RNA.

An additional step may include enriching labeled, capped RNA by immobilizing the labeled capped RNA on an affinity substrate and washing away the unlabeled RNA or where the label is an oligonucleotide, selectively amplifying the oligonucleotide labeled capped RNA. In one aspect, immobilized labeled capped RNA may be eluted from the affinity substrate prior to sequencing where the elution step is optional.

In one aspect, the RNA in the preparation comprises a naturally capped RNA and prior to performing the method above, the naturally capped RNA is combined with a decapping enzyme for removing the cap wherein the uncapped RNA has a 5' terminal diphosphate or triphosphate.

Examples of capping enzymes include *Vaccinia* capping enzyme (VCE) (New England Biolabs, Ipswich, MA), a Bluetongue Virus capping enzyme, a *Chlorella* Virus capping enzyme, and a S. *cerevisiae* capping enzyme. In one aspect, the label such as a receptor binding small molecule on the 5' labeled capped RNA is capable of targeting the 5' labeled capped RNA to cells *in vivo.* In another aspect, the 5' capped labeled RNA is capable of being detected in a complex environment by means of the label such as a fluorescent label in vivo or in vitro.

In one aspect, the invention provides a preparation or kit as further defined in the claims, which comprises a capping enzyme and a compound represented by Formula (I): 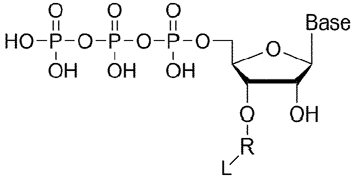 or a salt thereof, wherein: Base is a purine or a pyrimidine, R is a linker, wherein the linker is a straight or branched chain alkyene group with 1 to 300 carbon atoms and L is a label selected from the group consisting of an affinity label, a detection label, a chemoselective group, an oligonucleotide, and a combination thereof.

In another aspect, a kit is provided that includes the capping enzyme, a compound and instructions for selective labeling of RNA having 5' diphosphate or triphosphate with a labeled modified nucleotide as described above and optionally enriching for the same. The kit may further include an affinity matrix suitable for binding the labeled RNA.

In one aspect, the invention provides a method for enriching for prokaryotic non-ribosomal RNA in a mixture comprising eukaryotic and prokaryotic RNA, as further defined in the claims. The method includes: (a) combining a mixture of RNA comprising eukaryotic and prokaryotic RNA with a compound represented by Formula (I): 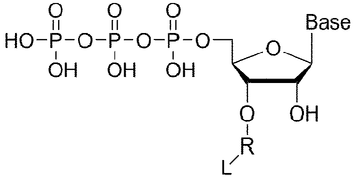 or a salt thereof, wherein: Base is a purine or a pyrimidine, R is a linker, and L is a label selected from the group consisting of an affinity label, a detection label, a chemoselective group, an oligonucleotide, and a combination thereof; in the presence of a capping enzyme, so as to form 5' capped labeled prokaryotic non-ribosomal RNA; (b) immobilizing the 5' capped labeled RNA; and (c) removing unreacted RNA.

In one aspect, the method further includes sequencing the 5' capped labeled RNA. In another aspect, the sequences are compiled into a transcriptome for a single organism or cell or a metatranscriptome for a plurality of different organisms or cells.

In one aspect, the invention provides a method for determining transcriptional start sites (TSS) of RNA, that includes: (a) obtaining total RNA from prokaryotic cells or eukaryotic cells or a mixture of eukaryotic and prokaryotic cells; (b) capping uncapped or decapped RNA having a 5' diphosphorylated or 5' tri-phosphorylated end from the total RNA with modified labeled nucleotide represented by Formula (I): 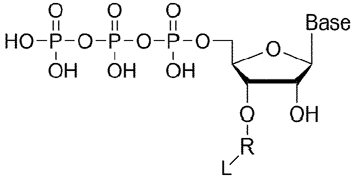 or a salt thereof, wherein: Base is a purine or a pyrimidine, R is a linker, and L is a label selected from the group consisting of an affinity label, a detection label, a chemoselective group, an oligonucleotide, and a combination thereof; in the presence of a capping enzyme, thereby forming 5' capped labeled RNA; and (c) sequencing the 5' capped labeled RNA or hybridizing the 5' capped RNA to a probe so as to determine the TSS of RNA.

An example of uncapped eukaryotic RNA with a 5' diphosphate or 5' triphosphate is nascent eukaryotic RNA. An example of uncapped prokaryotic RNA with a 5' diphosphate or triphosphate is mRNA and small RNAs.

In one aspect, 5' capped labeled RNA is fragmented prior to sequencing. In another aspect, the immobilized 5' capped labeled RNA is eluted and may be sequenced or hybridized to a probe for identifying the immobilized 5' capped labeled RNA wherein the step of hybridizing occurs when the RNA is immobilized or after the RNA is eluted from a matrix. In another aspect, the RNA sequences or the identified RNAs are compiled into a transcriptome or metatranscriptome. In another aspect, the RNA sequences or the identified RNAs are sequence specific markers and/or TSS and may be available at single base resolution. The sequence specific markers may constitute a signature profile.

In general, in one aspect, a method is provided for selectively binding a target RNA in a RNA population to a matrix, wherein the target RNA is characterized by a 5' triphosphate or 5' diphosphate that has been capped with a labeled modified nucleotide such as a compound as described above, the capped target RNA having a binding affinity to a matrix; and eluting the target RNA from the matrix to form an at least twofold or threefold or fourfold or fivefold enriched preparation of the target RNA when the representation of the eluted target RNA is compared to the representation of the target RNA in the RNA population. In one aspect, the eluted oligonucleotides comprise TSS. The TSS may be identified with single base resolution. In another aspect, the eluted oligonucleotides are sequenced to obtain a 5' sequence specific markers. In another aspect, the oligonucleotides which may be immobilized on the matrix or eluted into solution may be hybridized to probes, for example, in an array.

In one aspect, the RNA population includes capped RNA and/or uncapped RNA with or without a 5' triphosphate or 5' diphosphate. In another aspect, the method comprises fragmenting the population of RNA or the target RNA into oligonucleotides prior to capping or after capping with a labeled modified nucleotide for binding labeled capped fragmented RNA selectively to the matrix. For example, the fragmented RNA may have a length in the range of 8-800 nucleotides. In another aspect, the 3' phosphate on the fragmented capped labeled RNA is removed with a kinase.

In one aspect, the target RNA may be a prokaryotic transcriptome, a metatranscriptome for example from a microbiome or from a eukaryotic tissue sample, a nascent eukaryotic RNA and/or eukaryotic mRNA.

In one aspect, the eluted enriched target RNA is sequenced and the sequencing reads are quantified. In another aspect, the 5' TSS in the target RNA are quantified to obtain sequence specific markers for the RNA population. In one aspect, the sequence specific markers may be correlated with a phenotype of a eukaryotic host or a complex mixed population of microbes. In one aspect, target RNA with a 5' triphosphate or 5' diphosphate is labeled with (i) desthiobiotin or a derivative thereof to form a cap for binding reversibly to the matrix; or (ii) an oligonucleotide to form a cap for cap jumping and selective adapter dependent amplification. In a further aspect, biotin may be added for eluting the target RNA from the matrix.

Another aspect of the method includes decapping any capped RNA in the RNA population for recapping with a labeled modified nucleotide. Adapters may be added to the decapped ends of the eluted RNA for reverse transcribing to DNA and amplifying the DNA prior to sequencing. After sequencing, TSS may be obtained at single base resolution

In one aspect, the invention provides a method of selectively labeling uncapped RNA having a 5' tri-phosphate or 5' di-phosphate in a population of RNAs, comprising: combining the population of RNAs with a labeled modified nucleotide represented by Formula (I): 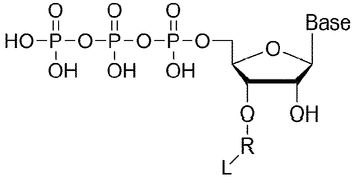 or a salt thereof, wherein: Base is a purine or a pyrimidine, R is a linker, and L is a label selected from the group consisting of an affinity label, a detection label, a chemoselective group, an oligonucleotide, and a combination thereof; so as to convert the uncapped RNA into selectively 5' labelled capped RNA; wherein the RNA is fragmented into oligonucleotides before or after selective labelling.

In one aspect, the fragmented RNA have a length in the range of 5-1000 nucleotides preferably 8-800 nucleotides or 10-500 nucleotides. In another aspect, the labeled oligonucleotides include TSS. In another aspect, the labeled oligonucleotides are sequenced to obtain 5' sequence specific markers. Sequencing of the oligonucleotides can provide single base resolution. Sequence specific markers can be assembled into a signature profile for a transcriptome or metatranscriptome and can then be correlated with a phenotype of a eukaryotic or prokaryotic cell or cells.

In another aspect, the eluted oligonucleotides are quantified by obtaining sequencing reads for each oligonucleotide. The label associated with the modified nucleotide may be desthiobiotin-GTP or a derivative thereof.

Target RNA having a labeled modified nucleotide at the 5' end can be distinguished in a mixture of molecules by means of the label on the modified nucleotide. A suitable label can be selected so as to achieve one or more of the following: enrichment of a target RNA, for example using desthiobiotin; selective amplification of a target RNA, for example using an oligonucleotide; labeling of a target RNA for example using a fluorescent label; sequencing of a target RNA after enrichment; stabilization of a target RNA for example by protection against enzyme digestion; or during *in vivo* administration of a target RNA; and targeted delivery of an RNA *in vivo.*

### BRIEF DESCRIPTION OF THE FIGURES

The figures and drawings are intended to illustrate one or more versions of the compositions and/or methods described herein. Unless stated otherwise, these are not intended to be limiting for the purpose of interpreting the scope of any claims.

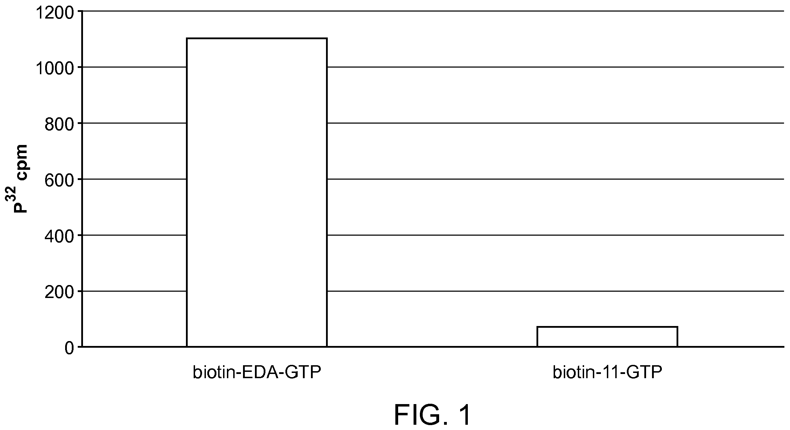

FIG. 1 is a histogram that contrasts the label position in the ribose moiety versus the purine base of a labeled compound for RNA capping. Biotin-EDA-GTP (Jena Biosciences, Germany) has biotin attached as a mixture of the 2' and 3' OH positions on the ribose ring. Biotin-11-GTP (Perkin Elmer, Waltham, MA) has biotin covalently linked to the guanine base. As described in Example 1, the RNA that was reacted with 2'/3' biotin EDA GTP was selectively bound to streptavidin as compared to transcript reacted with biotin-11-GTP. For these purposes labeling a position on the nucleotide base is much less effective than labeling a position on the ribose.
FIGS. 2A-2B shows the chemical structures of two different labeled mononucleotides. FIG. 2A is the chemical structure of purified 3'-desthiobiotin-TEG-guanosine 5' triphosphate with a linker attached to the oxygen at the 3' position of the ribose.
FIG. 2B is the chemical structure of 2'-desthiobiotin-TEG-guanosine 5' triphosphate with a linker attached to the oxygen at the 2' position of the ribose.
FIGS. 2C-2D shows an oligonucleotide modified nucleotide for capping and capped enzyme product.

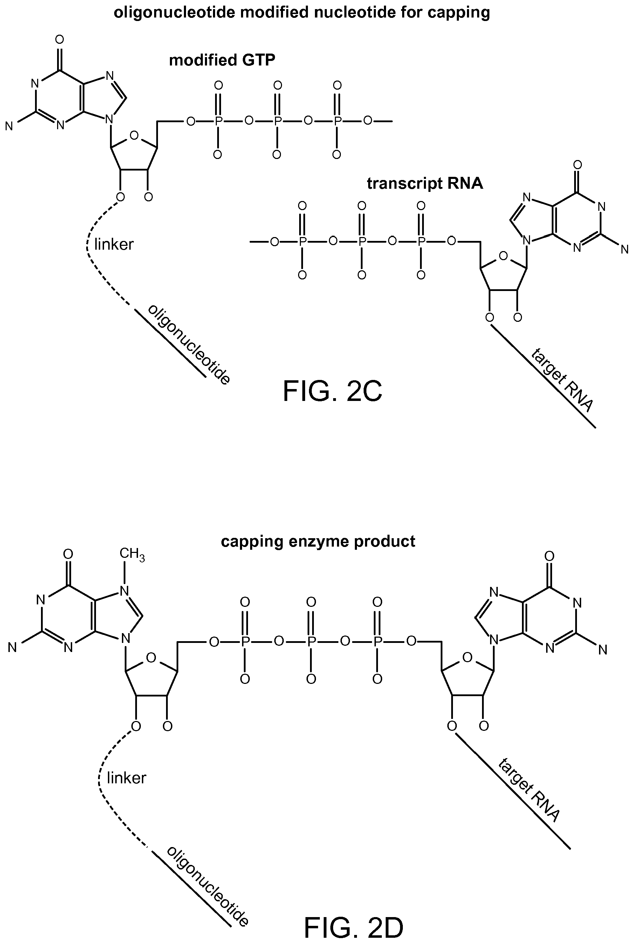

FIG. 2C shows labeled modified nucleotide and target transcript RNA where the label is an oligonucleotide.
FIG. 2D shows the reaction product of labeled modified nucleotide and target transcript RNA where the label is an oligonucleotide.

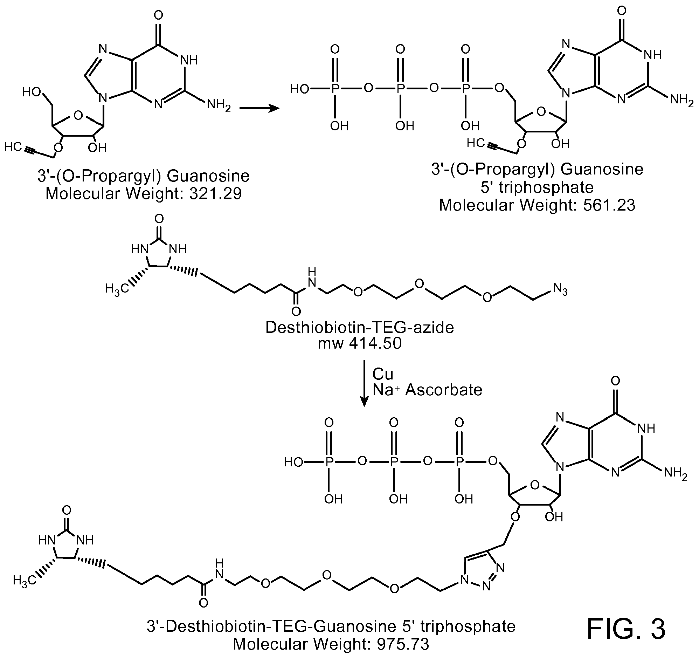

FIG. 3 shows a pathway for the chemical synthesis of 3' desthiobiotin-GTP.

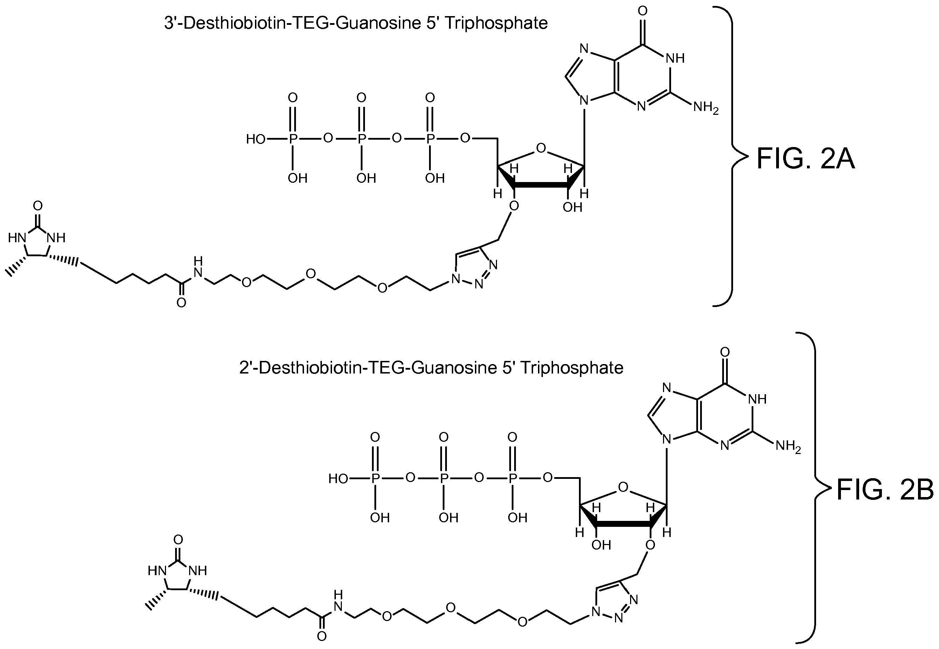

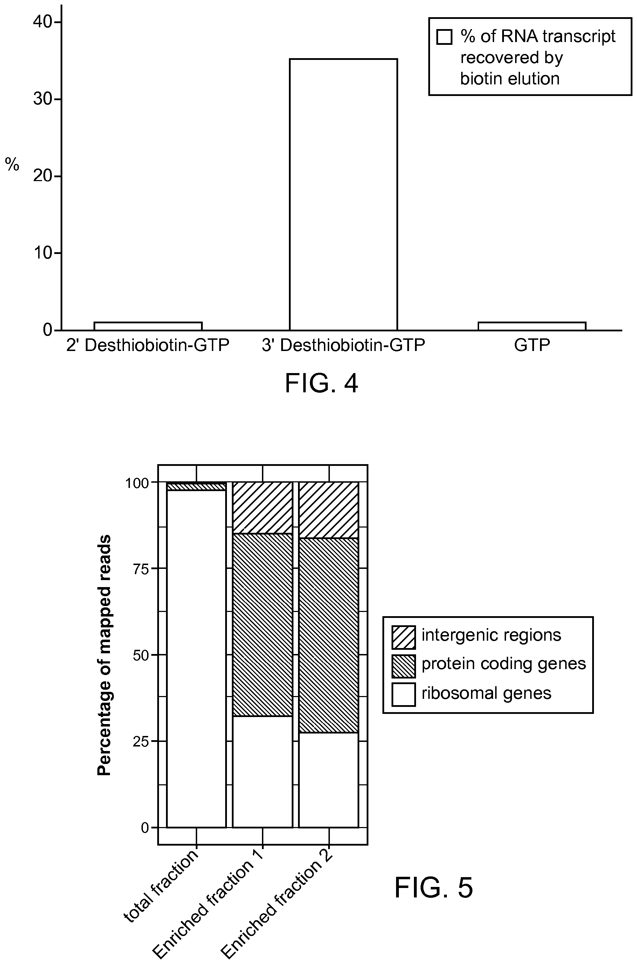

FIG. 4 shows results for capping of uncapped RNA with three different modified labeled nucleotides: 2' desthiobiotin-TEG-guanosine 5' triphosphate (2' desthiobiotin-GTP), 3' desthiobiotin-TEG-guanosine 5' triphosphate (3' desthiobiotin-GTP) and an unlabeled GTP control. The histogram shows that only RNA capped with 3' desthiobiotin-TEG-GTP (shown in FIG. 2A) was recovered after binding to streptavidin. In contrast, in the presence of a capping enzyme, RNA reacted with a mononucleotide, in which the desthiobiotin label was attached via a linker to the 2' hydroxyl group on the ribose moiety of GTP as described in Example 3, was surprisingly no better than control GTP for recovery.
FIG. 5 shows the distribution of reads mapping to intergenic regions, protein coding regions and ribosomal genes for total RNA, and two enriched fractions. Significantly, the mapped reads corresponding to ribosomal RNA is dramatically reduced in the enriched samples. Whereas 70 to 75% of the mapped reads from the enriched fractions corresponds to non-ribosomal RNA increased from about 5% in the total RNA sample. The y-axis is percentage of mapped reads. This data was obtained from libraries made from the total and enriched RNA using RNA SEQ (NEBNext® Ultra™ Directional RNA Library Prep Kit for Illumina®, New England Biolabs, Ipswich, MA) which was then sequenced in the Illumina miSEQ® (Illumina, San Diego, CA) as described in Example 16. Reads were mapped to the *E.coli* genome (U00096.2). Ribosomal genes, intergenic regions and protein coding genes were also defined by the NCBI annotation U00096.2

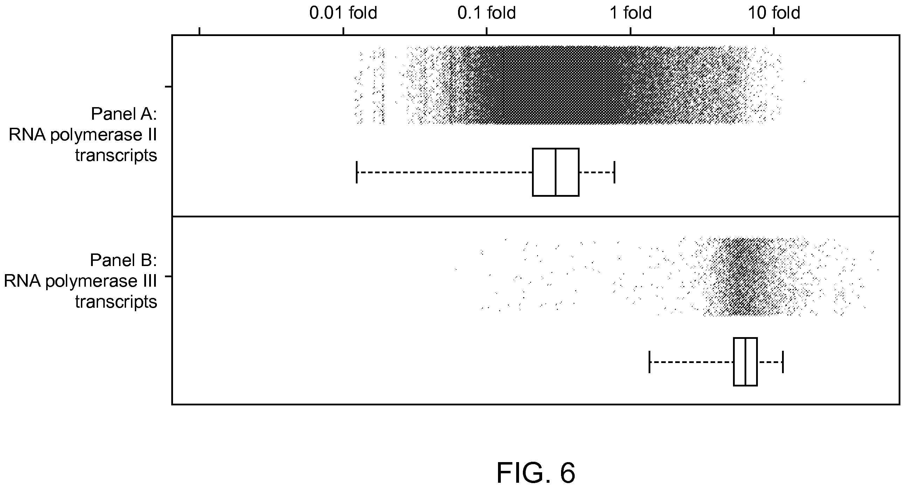

FIG. 6 shows a comparison of the distribution of points in a computer readout in which each point corresponds to the ratio between the relative amount of either an annotated pol II (panel A) or pol III RNA transcript (panel B) in the enriched versus total fraction. A majority of the pol III RNA transcripts were enriched at least 5 to 10 fold, whereas a majority of the pol II RNA transcripts were depleted demonstrating that Pol III transcripts were substrates for the modified labeled GTP, whereas the Pol II transcripts were not substrates.

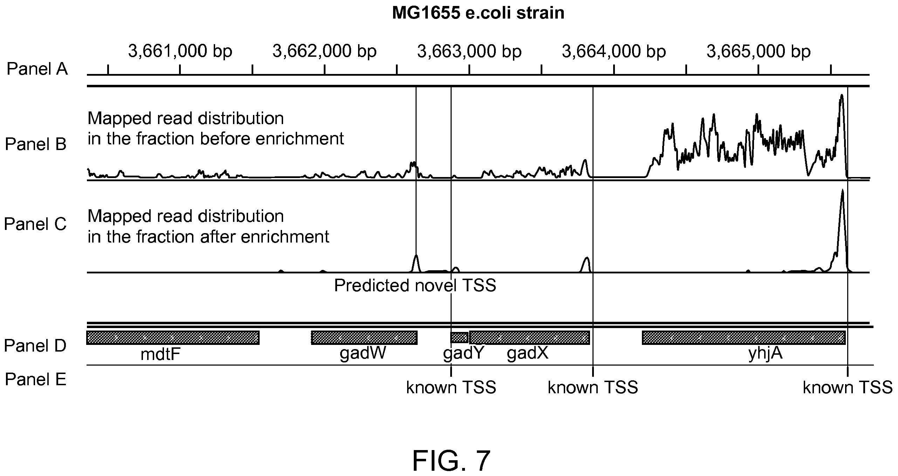

FIG. 7 provides a comparison of results for enriched and non-enriched 5' capped labeled prokaryotic RNA treated as described in Example 8. The total RNA was fragmented to about 200 nucleotide lengths prior to enrichment and then sequenced to identify TSS. In this example, TSS are detected at 3,662,888 bp, 3,663,865 bp and also at about 3,665,632 bp (U00096.2 genome). Panel (A) shows genomic region. Panel (B) shows mapped read distribution before enrichment. Panel (C) shows mapped read distribution in the fraction after enrichment showing peaks corresponding to three predicted TSS and a novel TSS. Panel (D) shows the location of genes to which the reads match. Panel (E) shows annotated TSS for the genes shown in (d) and shows that the enriched RNA shows a novel TSS at approximately 3662660.

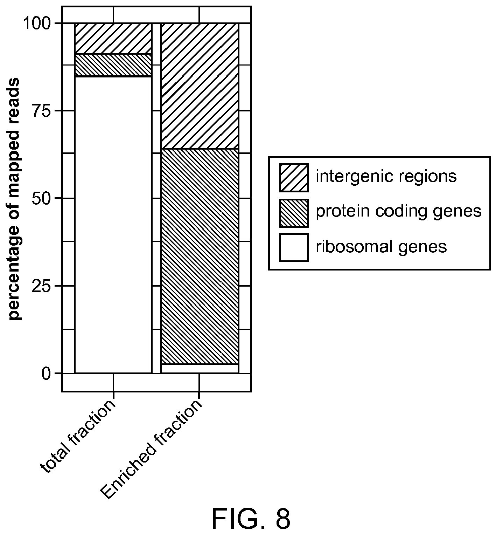

FIG. 8 shows the distribution of reads mapping to intergenic regions, protein coding regions and ribosomal genes for total RNA, and enriched RNA. Significantly, the mapped reads attributed to ribosomal genes is dramatically reduced in the enriched fraction from about 80% to about less than 5%. RNA mapping collectively in protein coding regions and intergenic regions increased from about 5% to greater than 95%. The y-axis is percentage of mapped reads. This data was obtained using NEBNext Small RNA Library Prep, (New England Biolabs, Ipswich, MA) to make libraries from the total and enriched RNA which was then sequenced in the Illumina miSEQ. Reads were mapped to the *E.coli* genome (U00096.2). Ribosomal genes, intergenic regions and protein coding genes were also defined by the NCBI annotation U00096.2

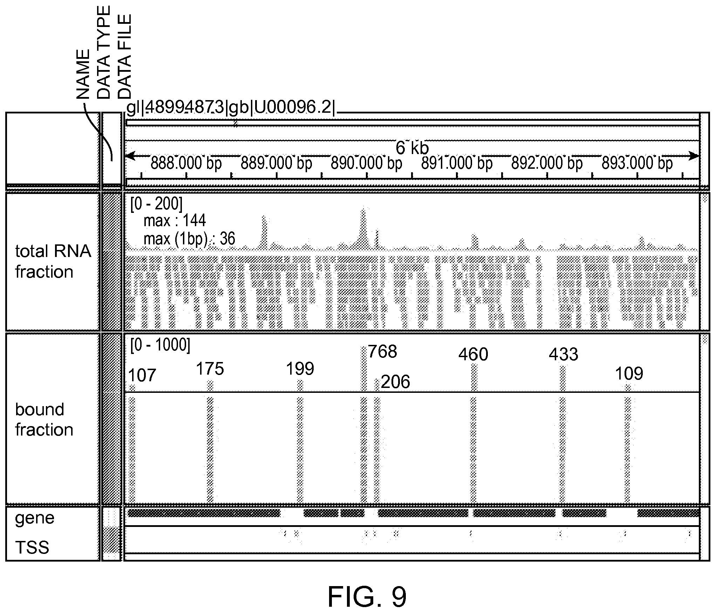

FIG. 9 shows that enrichment of non-ribosomal RNA and use of a small RNA library preparation for the samples results in sequencing reads positioned at TSS with single base pair resolution. Panel "total RNA fraction" shows individual read positions corresponding to positions distributed along the entire stretch of the genome fragment by forming an RNA library of total RNA. Panel "bound fraction", shows sequencing reads in the enriched fraction corresponding to TSS with single base resolution. Panel "gene" shows the position of genes in the genome fragment.

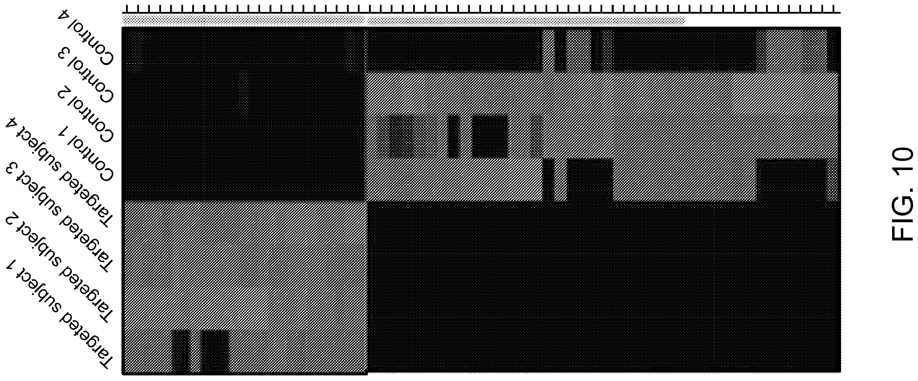

FIG. 10 shows a heat map from a subset of the microbiome signatures in 8 different samples, 4 controls and 4 treated subjects. Black corresponds to substantially no read representation whereas degrees of grey show a high degree of representation. The sequence signatures differentiate the control from the treated state. The horizontal bars along the right side vertical axis correspond to individual sequence specific markers.

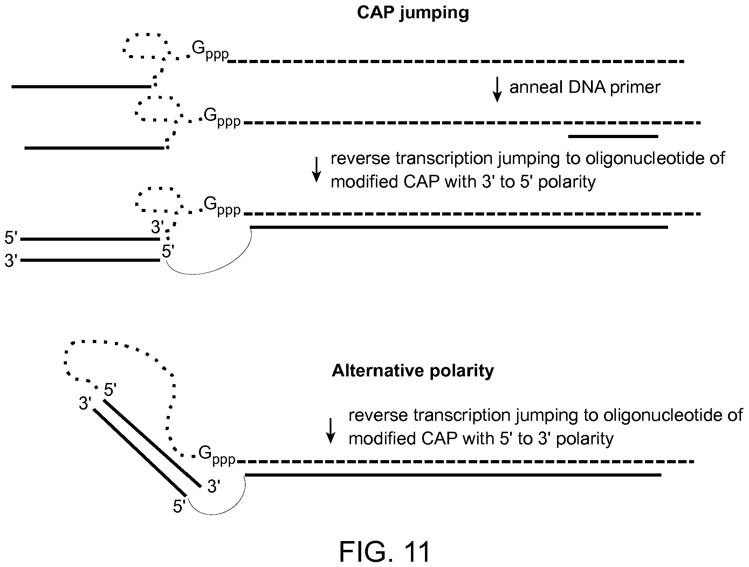

FIG. 11 shows cap jumping for selective amplification of a target RNA.

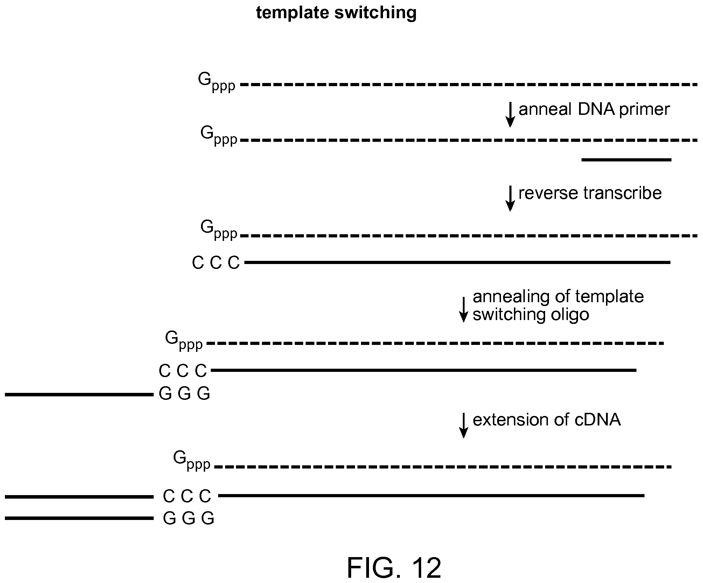

FIG. 12 shows template switching.

### DETAILED DESCRIPTION

The problem of sequencing target RNA species in prokaryotes has been made more difficult by the presence of a large amount of ribosomal RNA which varies in sequence between strains and other contaminating polynucleotides. Existing depletion techniques have a disadvantage in that some sequence specific to ribosomal RNA must be known to prepare suitable labeled oligonucleotides (Ribo Minus™, Life Technologies, Grand Island, NY).

Embodiments described herein are directed to enrichment of target RNAs and not depletion of specific contaminants. In this approach, the target RNA becomes immobilized and the unwanted RNA is removed by washing. An advantage of enrichment over depletion is that for enrichment, the removal of unwanted molecules is more effective and comprehensive than occurs through depletion which actually targets specific contaminants but may not remove all contaminants of a single species and none of the species of contaminant that is not targeted. When the target RNA is in low abundance, enrichment of the target RNA has the advantage of obtaining much larger amount of material that can be used for further analysis. Although not required, enrichment methods and depletion methods may be used sequentially.

In order for enrichment to be successfully accomplished, the target RNA should be efficiently recognized. Here this is achieved by labeling only those RNA molecules with a 5' tri-phosphate or di-phosphate regardless of its sequence or size. This is accomplished by using a modified labeled nucleotide and a capping enzyme. It has been shown here that capping enzymes such as VCE is capable of using a labeled modified nucleotide as a substrate when the modified nucleotide has a linker and a label.

To form the modified nucleotide, the specific location on the ribose for attaching the linker and label enables efficient capping. A modified nucleotide carrying a biotin label where the linker and label substitute for the hydrogen on the oxygen at carbon 3 of the ribose of the nucleotide is recognized by the capping enzyme and attached as a cap to the RNA. Whereas, if the same linker and label replaces the hydrogen on the oxygen at carbon 2 of the ribose of the nucleotide, the capping enzyme does not efficiently attach the labeled modified nucleotide to the RNA (see FIG. 2A-B, FIG. 3).

The capping enzyme is observed to be tolerant of the type of linker and label providing the position on the ribose is maintained. For example, no significant effect on capping efficiency is seen (see for example a EDA linker or a TEG linker). Similarly no significant effect on capping efficiency was observed when the label was varied (see for example, biotin and desthiobiotin) (see for example FIG. 1 and FIG. 4).

Embodiments provide uses for the enrichment methods. These include:
(a) Expression profiling of organisms in varying conditions by obtaining sequences of RNA populations excluding ribosomal RNAs. In prokaryotes, mRNA has a 5' triphosphate where eukaryotic mRNA is capped. However, eukaryotic mRNA can be decapped with a 5' deadenylase as described below and then recapped with labeled modified nucleotides for enrichment from eukaryotic ribosomal RNA. Ribosomal RNA has a 5' monophosphate and is therefore not amenable to decapping and recapping whereas eukaryotic mRNA is amenable to decapping and recapping. This method is particularly useful where the sequence of mRNAs may be unknown prior to analysis. This approach can reveal the presence, absence and biome characteristics of endosymbionts and/or parasites in a host.
(b) Transcriptomics of individual organisms involves analyzing all or a specific subset of non-ribosomal RNA species after enrichment as described herein. Meta-transcriptomics of mixed populations of cells such as may be found in a tissue or in a microbiome or environmental samples can also be determined after enrichment and removal of ribosomal RNA. Optionally a molecular signature of the meta-transcriptome can be obtained by digital gene expression profiling. In one example, where a microbial population in the context of a eukaryotic organism is analyzed, a small amount of prokaryotic mRNA of interest can be enriched while the large population of non-target RNAs include ribosomal RNAs (rRNA), transfer RNA (tRNA) and other so-called "house-keeping RNA" in addition to eukaryotic mRNA, rRNA and tRNAs can be removed.
   By way of illustration, example 4 describes enrichment of mRNA from *E. coli* from a mixture of total human RNA and total *E. coli* RNA. Universal Human Reference (UHR) RNA (Agilent) was mixed with total *E. coli* RNA and incubated with 3'desthiobiotin-TEG-GTP and VCE. An aliquot of total RNA was saved while the remainder was adsorbed to streptavidin beads. The beads were washed and the captured RNA was eluted using biotin. Barcoded Libraries were made using the NEBNext Ultra Directional RNA Library Prep Kit for Illumina as described by the manufacturer for both the total and enriched RNA fractions and sequenced on the Illumina MiSeq. Reads were mapped to a composite *E.coli* / human genome using STAR: ultrafast universal RNA-seq aligner (Dobin et al, Bioinformatics, doi: 10.1093/bioinformatics/bts635 (2012)).
(c) Viral RNA analysis. Virus RNA enrichment can be used for detection of virus and virus load and intracellular variants in eukaryotes for determining drug resistance, antigenic determinants, etc.
(d) Identification of TSS can be ascertained by fragmenting RNA before or after capping with modified labeled nucleotides. Only the 5' end fragment becomes immobilized and the remainder of the RNA including ribosomal RNA, non-5' terminal fragments and other 5' non triphosphorylated will be washed away. The sequencing burden is then much reduced allowing for simultaneous sequencing of mixed populations of prokaryotes (meta-transcriptome analysis) or obtaining reads of a greater number of samples from a single population (transcriptome analysis). Novel TSS in prokaryote transcriptomes were identified which had not been previously detected using alternative less sensitive methods.
   RNA SEQ methods that utilize RNA fragmentation and random priming followed by first and second strand cDNA synthesis prior to adapter ligation and PCR amplification resulted in peaks corresponding to TSS (see for example FIG. 7) although the precise start site varied within a limited nucleotide range. Surprisingly, substitution of RNA seq methods (NEB Next Ultra Directional RNA library Prep kit) with small fragment libraries utilizing adapter ligation to 3' and 5' ends of the RNA followed by first strand cDNA synthesis only and then PCR enrichment (NEBNext Small Library Prep Kit) provided single base resolution at the TSS (FIG. 9).
   Embodiments of the enrichment method enables the characterization of populations of prokaryotes such as occur in the microbiome using TSS signatures. This is an alternative to whole metatranscriptome sequencing and species identification. TSS signatures contain a much reduced complexity of the data per individual transcriptome, providing important data on which and how much primary transcript RNA is produced in a prokaryotic or eukaryotic cell or cells in a selected environment. The TSS signatures in eukaryotes may be correlated to the transcriptome of an individual cell or tissue to provide markers of health and disease including cancer.
   In a diagnostic procedure to which this analysis may be applied, a biological fluid may be obtained from a subject that potentially contains unknown prokaryotic and host eukaryotic cells. In one embodiment, the total RNA is isolated from this sample is fragmented for example, to about 20-200 nucleotides although the fragments may be longer or shorter than this depending on the amount of discriminatory power desired.
   For analysis of the prokaryotic population, the 5' end of prokaryotic RNA is uncapped and 5'-triphosphorylated or 5'-diphosphorylated. These characteristics distinguish the 5' end of prokaryotic RNA from the 5' end of eukaryotic RNA and also ribosomal and transfer RNAs. By attaching a tag on the 5' end by for example desthiobiotin, only those fragments of RNA at the 5' end of the prokaryotic RNA are bound to a suitable solid surface through the tag. Unbound material is washed away and the bound material can be eluted in the case of desthiobiotin, in the presence of free biotin.
   The released RNA can then be decapped, ligated to adaptors, reverse transcribed, amplified and sequenced using methods known in the art or can be sequenced using high-throughput sequencing methods.
   The transcriptome or meta-transcriptome can be analyzed by means of quantitatively determining sequence specific markers (SSM) of the RNAs obtained from the entire population of RNAs. Quantification can be obtained by counting the number of identical SSM in the RNA enriched sample. The length of the signature may be determined by the diversity of the population to be analyzed and the discriminatory power that is desired. A particular organism may be recognized by a few or hundreds or thousands of SSM. The panel of SSM represents a simplified representation of a functional state of a transcriptome or meta-transcriptome for correlation with a phenotype. Correlations and associations can be achieved by analyzing signatures of both a population from healthy hosts and those with an altered phenotype to determine qualitative and quantitative variations in the amounts and types of RNA produced by prokaryotes in the host samples. This approach of RNA analysis obviates the need to identify, classify and optionally diagnose individual bacterial species or eukaryotic genomes.
(e) Identification of lagging strand oligonucleotide primer sequences which are generated by primase during DNA replication. For example to determine leading and lagging strands and to locate the origin of replication and whether this might change in response to varying factors.
(f) Identifying labeled RNAs *in vivo* or *in vitro* by imaging. Labels on the modified nucleotide may include fluorescent labels.
(g) Identifying the properties of selected stabilized RNAs. Labels on the modified nucleotide may include a stabilizing label.
(h) Identifying different types of RNA by sequential analysis. For example, a first enrichment of eukaryotic total RNA would separate nascent RNA from the remainder of the RNA which could be recovered in the eluent for a second enrichment procedure. The second enrichment step might be achieved after decapping with a cap specific enzyme such as *Vaccinia* D9 or D10. A subset of total mRNA, the decapped RNA, could then be capped using a modified nucleotide such as described herein. Alternatively, one or more enrichment steps might be accompanied by a prior art depletion step.

### TERMS

Unless defined otherwise, all technical and scientific terms used herein have the same meaning as commonly understood by one of ordinary skill in the pertinent art. Embodiments described herein may include one or more ranges of values (*e*.*g*., size, concentration, time, temperature). A range of values will be understood to include all values within the range, including subset(s) of values in the recited range, to a tenth of the unit of the lower limit unless the context clearly dictates otherwise.

As used herein, the articles "a", "an", and "the" relate equivalently to a meaning as singular or plural unless the context dictates otherwise.

As used herein and as conventionally understood by those in the relevant art, a "nucleotide" comprises a base, a sugar and one or more phosphate groups. The base (also referred to as a "nitrogenous base" or a "nucleobase") is typically a purine or pyrimidine. The sugar is typically a five-carbon ribose (as in ribonucleotides) or a 2-deoxyribose (as in deoxyribonucleotides), which is bound via a glycosidic linkage to the base. Nucleotides typically have one, two or three phosphate groups (mono-, di- or tri-phosphates). Generally, the phosphate groups form a chemical bond at the 5-carbon position of the sugar, although they can also bond at the 2 or 3-carbon positions of the sugar group. Cyclic nucleotides form when a phosphate group is bound to two hydroxyl groups on the sugar. A "nucleoside" comprises a nucleobase and sugar. A nucleotide can thus also be called a nucleoside mono-, di- or tri-phosphate.

"Signature" refers to a collection of sequence specific markers (SSM).

"Sequence specific markers" (SSM) refers to the 5' terminal nucleic acid sequence of RNA molecules.

"Biological sample" refers to a sample from an environment within or external to a biological organism that is composed eukaryotic and/or prokaryotic cells. Examples of biological samples include feces, skin, saliva, lesion, soil, and water, a sample of organisms from a fermentation vessel or other organisms evolved from an environmental constraint that results in adaptive evolution.

"Chemoselective group", refers to one of a pair of groups that selectively react with one another to form a covalent bond. Chemoselective functional groups of interest include, but are not limited to, thiols and maleimide or iodoacetamide, as well as groups that can react with one another via Click chemistry, e.g., azide and alkyne groups (e.g., cyclooctyne groups).

"Target RNA" refers to an RNA that has a 5' diphosphate or triphosphate or can be converted to an RNA with 5' diphosphate or triphosphate by decapping or by kinase.

"Distinguishing an RNA" refers to any of: enrichment, selective amplification, selective labeling, sequencing and selective protection from enzyme digestion.

### Labeled modified nucleotide compounds

The labeled mononucleotide compounds may be a ribonucleotide or a nucleoside triphosphate. As discussed in Example 3, the label may be positioned at the 3' hydroxyl (OH) position of the sugar ring in the nucleotide. In contrast, labeling of the ribose 2' hydroxyl group is not suitable for the methods as described herein. In an embodiment, the labeled mononucleotide is not methylated.

An example of a labeled mononucleotide is shown in Formula (I): 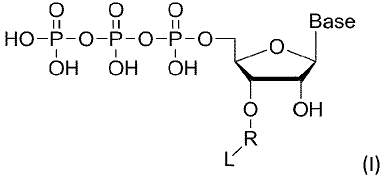 wherein the base is any nucleobase, R is a linker and L is a label.

In one embodiment, the base is a purine, pyrimidine, or analogs thereof, natural or synthetic. In an embodiment, the base is guanine or analog while in another embodiment, the base is inosine or analog thereof.

A linker R may be a covalent or electrovalent bond between the oxygen and a label. The linker R may be a flexible linker connecting a label L or a plurality of same or different labels to the oxygen in the 3' hydroxyl (OH) position of the ribose ring.

Linker molecules separating the label from the ribose may serve as steric spacers and do not necessarily have to be of defined length. Examples of suitable linkers may be selected from any of the hetero-bifunctional cross linking molecules described by Hermanson, Bioconjugate Techniques, 2nd Ed; Academic Press: London, Bioconjugate Reagents, pp 276-335 (2008).

The linker R can also increase the solubility of the compound in the appropriate solvent. The linkers used are chemically stable under the conditions of the actual application. The linker does not interfere with the mRNA capping reaction or with the detection of the label L, but may be constructed such as to be cleaved at some point in time after the reaction of the compound of Structural Formula (I) with the capping enzyme.

The linker R may be a straight or branched chain alkylene group with 1 to 300 carbon atoms, wherein optionally:
(a) one or more carbon atoms are replaced by oxygen, in particular wherein every third carbon atom is replaced by oxygen, *e*.*g*. a polyethyleneoxy group with 1 to 100 ethyleneoxy units;
(b) one or more carbon atoms are replaced by nitrogen carrying a hydrogen atom, and the adjacent carbon atoms are substituted by oxo, representing an amide function -NH-CO-;
(c) one or more carbon atoms are replaced by oxygen, and the adjacent carbon atoms are substituted by oxo, representing an ester function -O-CO-;
(d) the bond between two adjacent carbon atoms is a double or a triple bond, representing a function - CH=CH-or-C≡C-;
(e) one or more carbon atoms are replaced by a phenylene, a saturated or unsaturated cycloalkylene, a saturated or unsaturated bicycloalkylene, a divalent heteroaromatic or a divalent saturated or unsaturated heterocyclyl group;
(f) two adjacent carbon atoms are replaced by a disulfide linkage -S-S-;
or a combination of two or more, especially two or three, alkylene and/or modified alkylene groups as defined under (a) to (f) hereinbefore, optionally containing substituents.

A linker R may be a straight chain alkylene group with 1 to 25 carbon atoms or a straight chain polyethylene glycol group with 4 to 100 ethyleneoxy units, optionally attached to a -CH=CH- or -C=C- group. Further preferred is a straight chain alkylene group with 1 to 25 carbon atoms wherein carbon atoms are optionally replaced by an amide function -NH-CO-, and optionally carrying a photocleavable subunit, *e*.*g*. o-nitrophenyl. Further preferred are branched linkers comprising a polyethylene glycol group of 3 to 6 ethylene glycol units and alkylene groups wherein carbon atoms are replaced by amide bonds, and further carrying substituted amino and hydroxy functions. Other preferred branched linkers have dendritic (tree-like) structures wherein amine, carboxamide and/or ether functions replace carbon atoms of an alkylene group.

In one embodiment, any functionalized polyethylene glycol derivative may be used as a linker such as any of the pegylation products described in catalogs of Nanocs, Inc., Fisher Scientific, or VWR, Sigma-Aldrich Chemical.

A linker R may be a straight chain alkylene group of 2 to 40 carbon atoms optionally substituted by oxo wherein one or two carbon atoms are replaced by nitrogen and 0 to 12 carbon atoms are replaced by oxygen. For example, the linker R is a straight chain alkylene group of 2 to 10 carbon atoms wherein one or two carbon atoms are replaced by nitrogen and one or two adjacent carbon atom are substituted by oxo, for example a linker -CH2-NH(C=O)- or -CH2-NH(C=O)-(CH2)5-NH-.

Substituents considered are *e.g.* lower alkyl, *e.g.* methyl, lower alkoxy, *e.g.* methoxy, lower acyloxy, *e.g.* acetoxy, or halogenyl, e.g. chloro.

Further substituents considered are e.g. those obtained when an α-amino acid, in particular a naturally occurring α-amino acid, is incorporated in the linker wherein carbon atoms are replaced by amide functions - NH-CO- as defined in (b) above. In such a linker, part of the carbon chain of the alkylene group is replaced by a group -(NH-CHX-CO)n- wherein n is between 1 and 100 and X represents a varying residue of an α-amino acid.

A further substituent is one which leads to a photocleavable linker R2, *e*.*g*. an o-nitrophenyl group. In particular this substituent o-nitrophenyl is located at a carbon atom adjacent to an amide bond, *e*.*g*. in a group - NH-CO-CH2-CH(o-nitrophenyl)-NH-CO-, or as a substituent in a polyethylene glycol chain, *e*.*g*. in a group -O-CH2-CH(o-nitro-phenyl)-O-. Other photocleavable linkers considered are *e*.*g*. diazobenzene, phenacyl, alkoxybenzoin, benzylthioether and pivaloyl glycol derivatives.

A phenylene group replacing carbon atoms as defined under (e) above is *e.g*., 1,2-, 1,3-, or preferably 1,4-phenylene. In a particular embodiment, the phenylene group is further substituted by a nitro group, and, combined with other replacements as mentioned above under (a), (b), (c), (d), and (f), represents a photocleavable group, and is *e*.*g*. 4-nitro-1,3-phenylene, such as in -CO-NH-CH2-(4-nitro-)1,3-phenylene-CH(CH3)-O-CO-, or 2-methoxy-5-nitro-1,4-phenylene, such as in -CH2-O-(2-methoxy-5-nitro-)1,4-phenylene-CH(CH3)-O-, or 2-nitro-1,4-phenylene, such as in -CO-O-CH2-(2-nitro-)1,4-phenylene-CO-NH-. Other particular embodiments representing photocleavable linkers are *e*.*g*. -1,4-phenylene-CO-CH2-O-CO-CH2- (a phenacyl group), -1,4-phenylene-CH(OR)-CO-1,4-phenylene- (an alkoxybenzoin), or -3,5-dimethoxy-1,4-phenylene-CH2-O- (a dimethoxybenzyl moiety).

A saturated or unsaturated cycloalkylene group replacing carbon atoms as defined under (e) hereinbefore may be derived from cycloalkyl with 3 to 7 carbon atoms, preferably from cyclopentyl or cyclohexyl, and is *e.g.* 1,2- or 1,3-cyclopentylene, 1,2-, 1,3-, or preferably 1,4-cyclohexylene, or also 1,4-cyclohexylene being unsaturated *e.g*. in 1- or in 2-position.

A saturated or unsaturated bicycloalkylene group replacing carbon atoms as defined under (e) hereinbefore is derived from bicycloalkyl with 7 or 8 carbon atoms, and is *e*.*g*. bicycle [2.2.1] heptylene or bicyclo[2.2.2]octylene, preferably 1,4-bicyclo[2.2.1]-heptylene optionally unsaturated in 2-position or doubly unsaturated in 2- and 5-position, and 1,4-bicyclo[2.2.2]octylene optionally unsaturated in 2-position or doubly unsaturated in 2- and 5-position.

A divalent heteroaromatic group replacing carbon atoms as defined under (e) hereinbefore may, for example, include 1,2,3-triazole moiety, preferably 1,4-divalent 1,2,3-triazole. A divalent heteroaromatic group replacing carbon atoms as defined under (e) hereinbefore is *e*.*g*. triazolidene, preferably 1,4-triazolidene, or isoxazolidene, preferably 3,5-isoxazolidene. A divalent saturated or unsaturated heterocyclyl group replacing carbon atoms as defined under (e) hereinbefore is *e.g.* derived from an unsaturated heterocyclyl group, *e.g.* isoxazolidinene, preferably 3,5-isoxazolidinene, or a fully saturated heterocyclyl group with 3 to 12 atoms, 1 to 3 of which are heteroatoms selected from nitrogen, oxygen and sulfur, *e*.*g*. pyrrolidinediyl, piperidinediyl, tetrahydrofuranediyl, dioxanediyl, morpholinediyl or tetrahydrothiophenediyl, preferably 2,5-tetrahydrofuranediyl or 2,5-dioxanediyl. A particular heterocyclyl group considered is a saccharide moiety, *e*.*g*. an α- or β-furanosyl or α- or β-pyranosyl moiety.

The extension "-ylene" as opposed to "-yl" in for example "alkylene" as opposed to "alkyl" indicates that said for example "alkylene" is a divalent moiety connecting two moieties via two covalent bonds as opposed to being a monovalent group connected to one moiety via one covalent single bond in said for example "alkyl". The term "alkylene" therefore refers to a straight chain or branched, saturated or unsaturated hydrocarbon moiety; the term "heteroalkylene" as used herein refers to a straight chain or branched, saturated or unsaturated hydrocarbon moiety in which at least one carbon is replaced by a heteroatom; the term "arylene" as used herein refers to a carbocyclic aromatic moiety, which may consist of 1 or more rings fused together; the term "heteroarylene" as used herein refers to a carbocyclic aromatic moiety, which may consist of 1 or more rings fused together and wherein at least one carbon in one of the rings is replaced by a heteroatom; the term "cycloalkylene" as used herein refers to a saturated or unsaturated non-aromatic carbocycle moiety, which may consist of 1 or more rings fused together; the term "heterocycloalkylene" as used herein refers to a non-aromatic cyclic hydrocarbon moiety which may consist of 1 or more rings fused together and wherein at least one carbon in one of the rings is replaced by a heteroatom. Exemplary multivalent moieties include those examples given for the monovalent groups hereinabove in which one or more hydrogen atoms are removed.

Cyclic substructures in a linker R reduce the molecular flexibility as measured by the number of rotatable bonds within R, which leads to a better membrane permeation rate, important for all *in vivo* cell culture labeling applications.

A linker R may carry one or more same or different labels, *e*.*g*. 1 to 100 same or different labels, in particular 1 to 5, preferably one, two or three, in particular one or two same or different labels.

The label L may be selected from one or more of: an affinity label, a detection label, a reactive group and combinations thereof. In certain cases, the label may contain both an affinity label and a detection label.

Affinity labels are moieties that can be used to separate a molecule to which the affinity label is attached from other molecules that do not contain the affinity label. In many cases, an affinity label is a member of a specific binding pair, i.e. two molecules where one of the molecules through chemical or physical means specifically binds to the other molecule. The complementary member of the specific binding pair, which can be referred to herein as a "capture agent" may be immobilized (*e*.*g*., to a chromatography support, a bead or a planar surface) to produce an affinity chromatography support that specifically binds the affinity tag. In other words, an "affinity label" may bind to a "capture agent", where the affinity label specifically binds to the capture agent, thereby facilitating the separation of the molecule to which the affinity tag is attached from other molecules that do not contain the affinity label. Exemplary affinity tags include, but are not limited to, a biotin moiety (where the term "biotin moiety" is intended to refer to biotin and biotin analogs such as desthiobiotin, oxybiotin, 2'-iminobiotin, diaminobiotin, biotin sulfoxide, biocytin, *etc.,* that are able to bind to streptavidin with an affinity of at least 10-8M), avidin, streptavidin, protein A, maltose-binding protein, chitin binding domain, SNAP-tag® (New England Biolabs, Ipswich, MA) poly-histidine, HA-tag, c-myc tag, FLAG-tag, glutathione-S-transferase (GST), an epitope binding molecule such as an antibody, and polynucleotides that are capable of hybridizing to a substrate but excludes an alkyl group.

Exemplary detectable labels include, but are not limited to, optically detectable labels (*e*.*g*., fluorescent, chemiluminescent or colorimetric labels), radioactive labels, and spectroscopic labels such as a mass tag. Exemplary optically detectable labels include fluorescent labels such as xanthene dyes, *e*.*g*. fluorescein and rhodamine dyes, such as fluorescein isothiocyanate (FITC), 6 carboxyfluorescein (commonly known by the abbreviations FAM and F),6 carboxy-2',4',7',4,7-hexachlorofluorescein (HEX), 6 carboxy 4', 5' dichloro 2', 7' dimethoxyfluorescein (JOE or J), N,N,N',N' tetramethyl 6 carboxyrhodamine (TAMRA or T), 6 carboxy X rhodamine (ROX or R), 5 carboxyrhodamine 6G (R6G5 or G5), 6 carboxyrhodamine 6G (R6G6 or G6), and rhodamine 110; cyanine dyes, *e.g.* Cy3, Cy5 and Cy7 dyes; coumarins, *e.g* umbelliferone; benzimide dyes, *e.g.* Hoechst 33258; phenanthridine dyes, e.g. Texas Red; ethidium dyes; acridine dyes; carbazole dyes; phenoxazine dyes; porphyrin dyes; polymethine dyes, *e.g.* cyanine dyes such as Cy3, Cy5, *etc*; BODIPY dyes and quinoline dyes. Specific fluorophores of interest that are commonly used in some applications include: pyrene, coumarin, diethylaminocoumarin, FAM, fluorescein chlorotriazinyl, R110, eosin, JOE, R6G, tetramethylrhodamine, TAMRA, lissamine, ROX, napthofluorescein, Texas red, napthofluorescein, Cy3, Cy5, and FRET labels, *etc.*

In some embodiments, the label may be an oligoribonucleotide or an oligodeoxyribonucleotide, attached to the linker in either a 5' to 3' or a 3' to 5' orientation.

In some embodiments, the label is a chemoselective group that can be indirectly detected by reacting with a suitable reagent or substrate that contains one or more sites that covalently react with the reactive group.

A variety of different chemoselective groups may be used. For example, bis-NHS esters and maleimides (which react with amines and thiols, respectively), may be used. In other cases, the chemoselective group on the nucleoside may react with a reactive site on suitable reagent or substrate via click chemistry. In these embodiments, the nucleoside may contain an alkyne or azide group. Click chemistry, including azide-alkyne cycloaddition, is reviewed in a variety of publications including Kolb, et al., Angewandte Chemie International Edition 40: 2004-2021 (2001), Evans, Australian Journal of Chemistry, 60: 384-395 (2007) and Tornoe, Journal of Organic Chemistry, 67: 3057-3064 (2002).

The label can be detected directly or indirectly. Indirect detection means that the label is detected after interaction or reaction with another substrate or reagent. For example, through chemical conjugation, affinity partner binding, epitope binding with an antibody, substrate cleavage by an enzyme, donor-acceptor energy transmission (*e.g*., FRET), *etc.*

Label combinations for tandem affinity purification found in the literature were summarized in Li, Biotechnol. Appl. Biochem, 55:73-83 (2010). The table on page 74 of Li included the following where affinity tag/sequence or size (KDa)/Affinity matrix/Elution strategy is presented:

**Table 1**

| **Affinity tag** | **Sequence or size (KDa)** | **Affinity matrix** | **Elution strategy** |
|---|---|---|---|
| Z domain* | | IgG | IgG or low pH |
| CBP | KRRWKKNFIAVSAANRFKKISSSGAL (SEQ ID NO:4) | Calmodulin | 2 mM EGTA |
| His tag | HHHHHH (SEQ ID NO:5) | Ni2+, Co2+ | 150-500 mM imidazole |
| FLAG | DYKDDDDK (SEQ ID NO:6) | Antibody | FLAG peptide or low pH |
| HA | YPYDVPDYA (SEQ ID NO:7) | Antibody | HA peptide or low pH |
| Myc | EQKLISEEDL (SEQ ID NO:8) | Antibody | Low pH |
| V5 | GKPIPNPLLGLDST (SEQ ID NO:9) | Antibody | V5 peptide or low pH |
| Strep II | WSHPQFEK (SEQ ID NO:10) | StrepTactin | 2.5-5 mM desthiobiotin |
| SBP | MDEKTTGWRGGHVVEGLAGELEQLRARLEH HPQGQREP (SEQ ID NO:11) | Streptavidin | 2 mM biotin |
| S-peptide | KETAAAKFERQHMDS (SEQ ID NO:12) | S-protein | Denaturant or low pH |
| CBD | | Chitin | Thiol reagents or pH and temperature shift (when fused with intein) |
| GST | 26 | Glutathione | 10 mM reduced glutathione |
| MBP | 40 | Maltose | 10 mM maltose |

| | | | |
|---|---|---|---|
| *Z domain is a synthetic Fc-region-binding domain derived from the B domain of ProtA. | | | |

In some embodiments, the labeled modified nucleotide is 3'-O-(2-aminoethylcarbamoyl) (EDA)-biotin GTP. In some embodiments, the labeled nucleotide is 3'-desthiobiotin-triethylene glycol (TEG)-GTP. Example 2 describes the synthesis of 3' desthiobiotin-TEG-GTP. An advantageous feature of a desthiobiotin label is that it binds streptavidin less tightly than biotin and can be displaced by biotin ensuring that elution of enrich RNA is readily achieved.

In some embodiments the labels permit any variety of subsequent analysis of the labeled capped RNAs, including and without limitation isolation, purification, immobilization, identification, localization, amplification, and other such procedures known in the art.

The labeled modified nucleotide is described herein for use adding a label to targeted RNA molecules at the 5' end where the 5' end is characterized by a terminal di-phosphate or tri-phosphate. RNA with a 5' monophosphate or a cap (5'mGppp) are not amenable directly to labeling. However, if the 5'mG or 5'mGpp can be removed from a 5' capped RNA or one phosphate or two phosphates can be added to the 5' monophosphate, then these RNAs become substrates for labeled modified nucleotides.

The RNA from a biological sample generally include a diverse mixture of different species of capped and uncapped RNA; and may include non-RNA biological molecules such as any of those found in a cell lysate; and may additionally or alternatively include various natural or synthetic chemical formulations. In one embodiment, the RNA preparation does not include RNA polymerase.

The enrichment of selected species of RNAs for analysis (such as prokaryotic non-ribosomal RNA) minimizes problems of analysis associated with an overwhelming fraction of uninformative RNA (such as ribosomal RNA) that can mask a small minority of informative RNA.

The enrichment methods described herein are not dependent upon *in vitro* transcription, *in vitro* synthesis or cDNA intermediate synthesis methods from a genomic, cDNA, or other nucleotide sequence template.

The RNA may be obtained from one or more sources, including viruses, prokaryotic cells, eukaryotic or archaea cells or a mixture derived from tissue culture, biopsies, swabs, archived (such as paraffin embedded samples), the environment (air, water or land), or waste products.

The RNA preparations may include total cell RNA, size selected RNA, labeled RNA, and/or purified RNA. The RNA may be degraded or fragmented naturally or by means of standard techniques, such as mechanical shearing, enzymatic digestion, chemical cleavage, or sonication.

The RNA may include one or more of *in vitro* transcribed RNA, artificially synthesized RNA or obtained from RNA libraries. RNA may be obtained as RNA pol I transcripts, RNA pol II transcripts, RNA pol III transcripts, nascent RNA, primase, or prokaryotic RNA polymerase or any combination thereof.

The RNA may be a mixture of RNA species that include one or more of single stranded or double stranded RNAs. Single stranded RNAs include messenger RNA (mRNA), ribosomal RNA (rRNA), transfer RNAs (tRNAs), microRNAs (miRNAs), long non-coding RNAs (LNC-RNAs) that can be distinguished by the 5'ends. For example, prokaryotic and eukaryotic mature rRNA and tRNA have a 5'-monophosphate (5'P). Eukaryotic mRNA, with the exception of nascent transcripts and mature uncapped RNA, has a 5'-Gppp. Archaea, bacterial mRNA and small RNAs typically are uncapped and have a terminal 5' tri-phosphate (5'PPP). Naturally degraded RNA has a 5'-OH or 5'-P. A naturally occurring capped RNA has a 5'-m7Gppp.

An RNA preparation can be suitably prepared using any one or more of the methods described below to suitably optimize the RNA for enrichment. This may involve a capping reaction using a labeled compound described herein, and/or used to remove or deplete from the preparation, a population of RNA not intended for capping using a labeled compound as described herein. For example, a population of RNAs having a 5' tri- or di-phosphate, that is present in a complex mixture, can be enriched using various modifications to the enrichment protocol. For example fragmenting the RNA before enrichment will result in a library where only the 5' end of the RNA is analyzed which is useful for determining TSS. When the RNA is not fragmented before enrichment the resulting library will contain full length transcripts which are useful for transcriptome analysis. Furthermore after enrichment, the enriched RNAs can be prepared for RNA sequence analysis by different methods. When the RNA protocol for enrichment includes fragmentation and the preparation for sequencing includes decapping, followed by small RNA library preparation which includes 5' end ligation (NEBNext Small Library Prep), TSS to single base resolution emerge from the analysis. In another case, where the RNA protocol for enrichment includes fragmentation and the preparation for sequencing does not include decapping, standard RNASEQ libraries can be generated (NEBNext Ultra Directional RNA Library Prep) which can be analyzed for approximate positional TSS. In another case where the RNA was not fragmented, the RNA after enrichment can be left capped and standard RNASEQ libraries can be generated (NEBNext Ultra Directional RNA Library Prep) which can be analyzed to determine full length transcripts.

In embodiments of the invention, an uncapped RNA that has 5'-PPP or 5'-PP can be converted into a capped RNA by means of a capping enzyme, as defined in the claims. Capping reactions may involve more than one enzyme. Examples of different RNAs having 5'-PPP or 5'-PP include prokaryotic non-ribosomal RNA, uncapped eukaryotic nascent RNA, eukaryotic RNA polymerase III transcripts, eukaryotic RNA polymerase I transcripts, virus RNA, piwi RNA and primase RNA. The 5' end of RNA generated by degradation either has a monophosphate at the 5' end or a 5'OH. Prokaryotic ribosomal RNA has a 5' monophosphate whereas eukaryotic ribosomal RNA has a 5' monophosphate.

Examples of suitable capping enzymes include viral capping enzymes and their homologs such as VCE (see for example, Mao, et al., Journal of Biological Chemistry, 269:24472-24479 (1994), and Shuman, Journal of Biological Chemistry 265:11960-11966 (1990)) The VCE is composed of two proteins. The larger protein contains the active sites for all three activities (triphosphatase, guanylyltransferase and methylase) the smaller protein is bound to the larger one and is required in order for the methylase to be active. Other examples of capping enzymes include Bluetongue Virus capping enzyme (see for example Sutton, et al., Nat Struct Mol Biol 14: 449-451 (2007) and Ramadevi, et al. Proc Natl Acad Sci. USA 95:13537-13542 (1998)) and Chlorella Virus capping enzyme (see for example, Gong, et al., Journal of Biological Chemistry 277:15317-15324 (2002), Ho, et al., Journal of Virology, 70:6658-6664 (1996) and Ho, et al., Journal of Virology, 75:1744-1750 (2001) and yeast capping enzymes such as from S. *cerevisiae,* and related homologs (see for example, Steiger, et al., RNA, 9:231-238 (2003), Bougie, et al., Biochem J, 384:411-420 (2004) and Lima, et al., Cell, 99:533-543 (1999). The capping enzyme used herein includes a wild type amino acid sequence or variants or thereof having a sequence that is at least 90% identical, *e*.*g*., at least 95% identical at least 98% identical, or at least 99% identical to a wild type amino acid sequence (SEQ ID NO:1 and SEQ ID NO:2).

Conditions suitable for capping enzyme activity include those recommended by manufacturers of commercially available enzymes (see for example, New England Biolabs, Ipswich, MA) that are routine for those in the relevant art. For example, the enzymes are active in suitable buffers at temperature ranges of about 15°C to about 42°C, for example, about 37°C.

A capped RNA can be decapped to form an uncapped RNA having a 5' terminal monophosphate using enzymes for example *Vaccinia* decapping enzyme D9, *Vaccinia* decapping enzyme D10, human Dcp2, tobacco acid pyrophosphatase (TAP), and Nudt 16; RppH (see for example, US 8,486,666).

Uncapped substrate can be recapped with a labeled compound such as described herein. Any methods for removing the cap and leaving a 5' diphosphate or 5' triphosphate on the RNA will be suitable for subsequent capping reactions with a labeled mononucleotide using the methods described herein. For example, decapping can be achieved using an enzyme such as 5' deadenylase (*e.g*., *S. cerevisiae* 5' deadenylase (see US 8,486,666)), DcpS (*e.g*., human DcpS or S. *cerevisiae* DcpS) and the like.

RNA with a 5' monophosphate is not a substrate for a capping enzyme, unless the 5' monophosphate is converted to a 5' diphosphate or 5' triphosphate by adding phosphates with a kinase. An example of a suitable kinase is 5'-phosphate-polyribonucleotide kinase (Spencer, et al., PNAS, 75:4793-4797 (1978)). The kinase phosphorylates the 5' monophosphate RNA into 5' di- and/or 5' tri-phosphate RNA, which is then a suitable substrate for a capping enzyme to cap the RNA with a labeled mononucleotide as described herein.

However, if the phosphate groups are removed from the 5' terminus by for example, cleaving with an enzyme such as a phosphatase then uncapped RNA can no longer be capped. Phosphatases include enzymes that remove all phosphate groups leaving behind a 5' hydroxyl group on the RNA. Examples of such enzymes include calf intestine alkaline phosphatase (CIP), bacterial antarctic phosphatase, and shrimp alkaline phosphatase. Other phosphatases can cleave just the terminal phosphate groups to leave a monophosphate at the 5' terminus of RNA. Examples of such enzymes include 5' RNA polyphosphatase, RppH, apyrase and analogs, derivatives and related enzymes.

A composition is also provided. In certain embodiments, the composition may comprise: a) a compound, as described above, b) a capping enzyme, as described above, and c) uncapped RNA. In some embodiments, the composition may additionally comprise a substrate (e.g., beads or the like) comprising a group that binds to or reacts with the capture moiety of the compound. As would be recognized, the composition may be buffered and may contain other components, e.g., salt, divalent cations, etc., that are required by the enzyme.

Also provided by this disclosure is a kit for practicing the subject method, as described above. A subject kit may contain at least: a) a compound, as described above and b) a capping enzyme, as described above. In some embodiments, the kit may additionally comprise a substrate (e.g., beads or the like) comprising a group that binds to or reacts with the capture moiety of the compound.

In addition, the kit may also comprise reagents for performing the reaction, e.g., one or more buffers. The various components of the kit may be present in separate containers or certain compatible components may be pre-combined into a single container, as desired.

In addition to above-mentioned components, the subject kits may further include instructions for using the components of the kit to practice the subject methods, i.e., to provide instructions for sample analysis. The instructions for practicing the present method may be recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g., CD-ROM, diskette, etc. In yet other embodiments, the actual instructions are not present in the kit, but means for obtaining the instructions from a remote source, e.g., via the internet, are provided. An example of this embodiment is a kit that includes a web address where the instructions can be viewed and/or from which the instructions can be downloaded. As with the instructions, this means for obtaining the instructions is recorded on a suitable substrate.

RNA may be added to the reaction vessel containing these components and reacted accordingly. After enrichment, RNA can be subsequently manipulated as described below.

In some embodiments, the RNAs capped with a labeled mononucleotide using a capping enzyme can be subsequently manipulated. For example, the labeled capped RNAs can be isolated (captured, purified, enriched) by, for example affinity binding to a suitable matrix. Any suitable matrix can be used, such as and without limitation, a solid, semi-solid, or porous matrix. The matrix can be in any suitable form such as beads including magnetic beads, column, plate, or microfluidic device. Such matrices can be treated, adsorbed, affinity coated, with a binding reagent, ligand or labeling partner specific for binding the label on the mononucleotide. The matrix may be made of any suitable materials, including metal, polystyrene, glass, paper, protein or other biological or chemical reagent such as a polymer. Once bound to the matrix, the bound capped RNAs can be washed, eluted or otherwise isolated and optionally purified from the mixture for subsequent analysis as desired. Enrichment by immobilization on a matrix can be achieved at temperatures in the range of 25°C to 80°C, for example, 25°C to 75°C or 30°C to 60°C.

In some embodiments, the RNAs capped according to the methods described herein may be fragmented before or after capping. Such fragmenting reduces the sizes of the RNA to any desired length. For example, the RNA fragments can be around 10-10000 nucleotides in length, or ranges in between, e.g., 100-1000 nucleotides, 10-500 nucleotides, 3000-5000 nucleotides, or about 50, 100, 200, 250 nucleotides. Fragmenting can be achieved using standard techniques, including mechanical shearing, chemical, enzymatic digestion and sonication.

In some embodiments, the 3' ends of RNA can be reacted with T4 Polynucleotide Kinase (New England Biolabs, Ipswich, MA) in the absence of ATP, to remove 2'- 3' cyclic phosphate or 3' phosphate.

In some embodiments, the capped RNAs can be sequenced. Sequencing will not only identify the nucleotide sequence of the RNA and characterize it along with the population of other labeled capped RNAs if desired such as in microbiome analysis and expression profiling but sequencing can also pinpoint and identify the TSS sequence of RNAs. Sequencing can also identify nascent RNA that is newly transcribed. cDNA library preparation for Next-Generation sequencing can be done on the labeled capped RNAs using the NEBNext Ultra Directional RNA Library Prep Kit for Illumina or equivalent protocols.

The labeled capped RNAs can be directly ligated to adapters for preparing small RNA libraries (using the NEBNext Small RNA Library Prep Set for Illumina or equivalent protocol). In this case, the labeled capped RNAs may be decapped prior to ligation with an adapter or vector where necessary. Such a library will represent the specific population of RNAs capped with the labeled nucleotide as described herein (e.g., enriched capped mRNAs, uncapped RNAs, *etc*.)*.* Alternatively the labeled capped RNAs can be reverse transcribed using a template switching oligonucleotide (Luo, et. al., J. Virol., 64(9): 4321-4328 (1990); Zhu, et al., Biotechniques 30:892-897 (2001)) that enables introduction of a necessary priming sequence for the generation of DNA libraries for NextGen sequencing.

When the label of the capping nucleotide is composed of an oligonucleotide, cap jumping (Efimov, et al., Nucl. Acids Res., 29(22):4751-4759 (2001)) can be used to introduce the necessary priming sequence for the generation of DNA libraries for NextGen sequencing.

### EXAMPLES

### Example 1: RNA Capping Using Biotin-11-GTP or 2'/3' EDA-biotin-GTP: Comparison of the Label Position on the Nucleotide Base versus the Sugar Ring

A 300mer uniformly P³² labeled *in vitro* RNA transcript was incubated with VCE and VCE buffer (New England Biolabs, Ipswich, MA) and either biotin-11-GTP (label on the guanosine ring) or 2'/3' Biotin-EDA-GTP.

Ten microliter reaction volumes containing 1x VCE buffer, P³² uniformly labeled *in vitro* 300mer transcript RNA, 10 units of VCE and either 0.5mM EDA-biotin-GTP, or 0.1mM biotin-11-GTP were incubated at 37°C for 60 minutes. The RNA from the reaction mixes were then purified on MEGAclear™ (Life Technologies, Grand Island, NY) spin columns as directed by the manufacturer. 20 µl of each purified RNA (20% of the total volume) was mixed with 5 µl (5 µg) of ΦX174 DNA cleaved by Haelll (New England Biolabs, Ipswich, MA). This mix was mixed with 125 µl of hydrophilic streptavidin magnetic beads (New England Biolabs, Ipswich, MA) that had been prepared by washing with a wash buffer (0.4 ml of wash buffer: 20 mM Tris-HCL pH 7.5, 500 mM NaCl, 1 mM EDTA) and incubated for 10 minutes at room temperature. The beads were then washed to elute unbound material by washing consecutively with 100 µl wash buffer, 120 µl wash buffer, 120 µl wash buffer and 400 µl of wash buffer. The beads were resuspended in 200 µl of wash buffer and radioactivity retained by the beads was determined by Cherenkov counting.

The results in FIG. 1 show that when biotin-11-GTP was used in the capping reaction of a 5' tri-phosphorylated RNA, no significant binding of the RNA to streptavidin beads was observed. However, when biotin-EDA-GTP (a mixture of 2' and 3' adducts) was used, about 10 fold more binding was observed than for biotin-11-GTP.

### Example 2: Synthesis of 3' desthiobiotin-TEG-GTP

This example describes the method of synthesis of a novel labeled nucleotide. Desthiobiotin is characterized here by a linker (TEG) that is attached to the oxygen on the C3 of the ribose. The linker is in turn linked to a desthiobiotin label which in contrast to Biotin has the property of being capable of being eluted from streptavidin without additional enzymatic reactions but solely by the addition of Biotin.

Synthesis was initiated with 3'-(O-Propargyl) Guanosine (ChemGenes Corp. Wilmington, MA) followed by its conversion to 3'(O-Propargyl) Guanosine 5' Triphosphate via a one-pot, two step method (several published procedures). The 3'-(O-Propargyl) Guanosine 5' Triphosphate was then purified by both ion exchange chromatography and reverse phase HPLC. The isolated 3'(O-Propargyl) Guanosine 5' Triphosphate was converted to the 3'-Desthiobiotin-TEG-Guanosine 5' Triphosphate through the addition of Desthiobiotin-TEG-azide (Berry and Associates, Inc., Dexter, MI) using copper-mediated azide-alkyne cycloaddition ("Click chemistry", Kolb and Sharpless, Scripps Res. Inst and BaseClick, Tutzing, GmbH). Final isolation of the target compound was performed using reverse phase HPLC. The pathway described here is shown in FIG.3.

### Example 3: Selective specificity of the label position at 3' OH position versus the 2' OH in the ribose ring of the nucleotide for RNA capping

Because biotin-EDA-GTP is a mixture of 2' and 3' adducts, it was decided to synthesize pure forms of the desthiobiotin-TEG-GTP with 2' adducts or 3' adducts and to test which of these were effective for binding RNA to streptavidin via the desthiobiotin. 2' desthioBiotin-TEG-GTP and 3' desthioBiotin-TEG-GTP (3' desthiobiotin-GTP) shown in FIG. 2A and 2B were synthesized according to the protocol described in FIG. 3 and Example 2.

A 300mer uniformly P³² labeled *in vitro* T7 transcript was incubated with VCE in VCE buffer and either 2' desthiobiotin-TEG-GTP or 3' desthiobiotin-TEG-GTP, or unlabeled control GTP.

The different modified labeled nucleotides were tested as follows: 10 µl reaction volumes containing 1x VCE buffer, P³² uniformly labeled T7 *in vitro* 300mer transcript RNA, 10 units of VCE and either 0.5mM 2' desthiobiotin-TEG-GTP (made according to the protocol in FIG. 3 and Example 2, where 2' O-Propargyl Guanosine was substituted for 3' O-Propargyl Guanosine) or 3' desthiobiotin-TEG-GTP (made according to the protocol in FIG. 3 and Example 2), or GTP were incubated at 37°C for 2 hours. 5 µl of MspI-digested pBR322 DNA (New England Biolabs, Ipswich, MA) was added to the RNA that was then purified on MEGAclear spin columns as directed by manufacturer. 50 µl (50 % of the total volume) RNA was mixed with 50 µl of wash buffer 2 (10 mM Tris-HCI pH 7.5, 500 mM NaCl, 1mM EDTA). This mix was added to the hydrophilic streptavidin magnetic beads (New England Biolabs, Ipswich, MA) that had been previously prepared by washing 3 times with 400 µl of 10 mM Tris-HCI pH 7.5, 50 mM NaCl. The beads were incubated for 10 minutes at room temperature. The beads were then washed with 100 µl of 10 mM Tris-HCI pH 7.5, 500 mM NaCl, 1 mM EDTA, and three times with 400 µl of 10 mM Tris-HCI pH 7.5, 500 mM NaCl, 1 mM EDTA, to elute unbound material. The beads were the resuspended in 50 µl of 10 mM Tris-HCI pH 7.5, 0.5M NaCl, 1 mM EDTA and an additional 50 µl of 10 mM Tris-HCI pH 7.5, 500 mMNaCl, 1 mM EDTA containing 20 mM biotin. The beads were kept resuspended for 20 minutes at room temperature by occasional quick mixing. To determine if the RNA had been selectively captured by the beads and eluted with the biotin, the beads were collected on the side of the tube with a magnet and the 100 µl supernatant was collected and radioactivity determined by scintillation counting.

The results showed that the transcript that was reacted with 3' desthiobiotin-TEG GTP was selectively bound to the streptavidin beads and eluted with 10 mM biotin whereas 10 mM biotin elution resulted in little to no P³² when 2' desthiobiotin-TEG-GTP or GTP were used as substrates for the VCE reaction. The results are shown in FIG. 4.

In addition, 3' biotin-TEG-GTP was tested with VCE and a 27 nucleotide *in vitro* transcription RNA product. Polyacrylamide gel electrophoresis confirmed that the transcript was capped with 3'Biotin-TEG-GTP.

### Example 4: Enriching 5' triphosphorylated and 5' diphosphorylated prokaryotic RNA from a mixture of eukaryotic and prokaryotic RNA

5' tri-and di phosphorylated RNA from *E. coli* could be enriched from a mixture of total human RNA and total *E. coli* RNA. 6 µg of Universal Human Reference (UHR) RNA (Agilent, Santa Clara, CA) was mixed with 6 µg of total *E. coli RNA* (prepared from an *E. coli* culture with FastRNA PRO™ Blue Kit, MP Biomedical, Santa Ana, CA) in a 70 µl volume containing 1x VCE buffer, 0.5 mM 3'desthiobiotin-TEG-GTP and 60 units of VCE and incubated at 37°C for 2 hours. The resulting reaction was applied to a MEGAclear spin column and eluted with 100 µl of water. A ten microliter aliquot was saved as unenriched control. 50 µl of buffer 1 (20 mMTris-HCI pH 7.5 50 mM NaCl) was added to the remaining 90 µl and the total 140 µl solution was adsorbed to the hydrophilic streptavidin beads that had been previously washed with buffer 1. The beads were incubated at room temperature for 20 minutes and washed 4x with buffer 2 (20 mMTris-HCl, 500 mM NaCl, 1 mM EDTA). The beads were then suspended in 100 µl of buffer 1 containing 1 mM biotin and incubated at room temperature for 20 minutes with occasional mixing. The beads were collected on the side of the tube with a magnet and the 100 µl supernatant was collected. The resulting biotin eluted RNA product was isolated by use of MEGAclear™ Kit (Life Technologies, Grand Island, NY). The biotin eluted RNA (enriched RNA) and the total RNA were then concentrated by use of "RNA Clean and Concentrator™" (Zymo Research, California) in 10 µl of water. Both RNA samples were prepared for sequencing by using the NEBNext Ultra Directional RNA Library Prep Kit for Illumina as described by the manufacturer and sequenced on the Illumina MiSeq.

Sequencing reads were quality filtered and the adaptors were trimmed. The reads were mapped to a composite genome made of the human (hg19) and *E. coli* (U00096.2) genomes using STAR [STAR: ultrafast universal RNA-seq aligner. Dobin].

The results showed that the proportion of sequenced RNAs that did not have tri- and di phosphorylated RNA (including prokaryotic and eukaryotic ribosomal RNA sequences and eukaryotic mRNA) was much reduced in the enriched fraction compared to the total RNA sample (before enrichment). More specifically, the number of prokaryotic non-ribosomal RNA increased more than 4-fold after enrichment, and the relative quantity of reads of prokaryotic non-ribosomal RNA in the sample increased from about 10% before enrichment to around 50% after enrichment.

**Table 2: Analysis of Enrichment**

| Reads mapping to: | Before Enrichment | After Enrichment |
|---|---|---|
| *E. coli* non-ribosomal RNA (RNA with 5' tri-and di phosphates) | 2211386 | 9129621 |
| *E. coli* ribosomes (Bacterial rRNA) | 4800190 | 2467861 |
| Human RNA (Eukaryotic RNA not 5' tri-and di phosphates) | 14888634 | 6532592 |

### Example 5: Enriching 5' triphosphorylated and 5' diphosphorylated RNA from an E.coli lysate

Controls - *in vitro* synthesized Fluc RNA and Cluc RNA were prepared using T7 RNA polymerase (New England Biolabs, Ipswich, MA) to transcribe plasmid DNA containing either the Cluc gene (pCMV-Cluc 2 Control plasmid (New England Biolabs, Ipswich, MA)) or the Fluc genes (Fluc Control Template from T7 Quick high yield RNA synthesis kit (New England Biolabs, Ipswich, MA)). The Fluc transcript was further treated with VCE and GTP to convert it to m7G capped RNA.

6 µg of total *E. coli* RNA, 7 ng of Fluc (m7G capped)RNA and 12 ng of Cluc (5' triphosphate) RNA were incubated in a 70 µl reaction volume with 1 x VCE buffer, 0.5 mM 3'desthiobiotin-GTP and 60 units of VCE for 2 hours at 37°C. The product of the reaction was applied to a MEGAclear spin column and eluted with 100 µl of water. A 33 µl aliquot was saved as unenriched sample.

125 µl hydrophilic streptavidin magnetic beads were prewashed with 3 times with a first wash buffer (0.4 ml of 10 mM Tris-HCI pH 7.5, 1 mM EDTA, 0.5 M NaCl) and then 1 time with a second wash buffer (0.4 ml of 10 mM Tris-HCI pH 7.5, 1 mM EDTA, 0.05 M NaCl). The beads were then suspended in 95 µl of the second wash buffer containing 2 µl of murine RNAase inhibitor (80 units) (New England Biolabs, Ipswich, MA). 33 µl of the 3' desthiobiotin GTP treated RNA was added to the streptavidin bead preparation. The beads were incubated at room temperature for 20 minutes with occasional mixing to keep the beads resuspended. The beads were then washed 2 times with the first buffer (0.4 ml 10 mM Tris-HCl pH 7.5, 1 mM EDTA, 0.5 M NaCl) containing 8 µl of the murine RNAase Inhibitor (320 units) and 2 times with 0.4 ml 10 mM Tris-HCl pH 7.5, 1 mM EDTA buffer, containing 8 µl of murine RNAase Inhibitor. The beads were then suspended in 100 µl of the second wash buffer (10 mM Tris-HCl pH 7.5, 1 mM EDTA, 0.05M NaCl) containing 1 mM biotin and incubated at room temperature for 20 minutes with occasional mixing. The beads were collected on the side of the tube with a magnet and the 100 µl supernatant was collected. The resulting biotin eluted RNA product and unenriched aliquot were then concentrated by use of "RNA Clean and Concentrator" in 10 µl of water. The two RNA samples were prepared for sequencing by using the NEBNext Ultra Directional RNA Library Prep Kit for Illumina as described by the manufacturer and sequenced on the Illumina MiSeq.

Enrichment of Cluc from a mixture of known amounts of Cluc (5' triphosphate RNA) and Fluc (m7G capped) *in vitro* synthesized transcripts was determined. Sequencing reads were quality filtered and the adaptors were trimmed. The reads were mapped to *E.coli* (U00096.2) genome and CLUC and FLUC transcript sequences using BWA [PMID:19451168]. An enrichment of greater than about 10 fold enrichment of the tri-phosphorylated RNA (CLuc) compared with the G-capped RNA (Fluc) and unenriched CLuc was observed.

Enrichment of non-ribosomal prokaryotic RNA from an *E.coli* lysate was greater than 8 fold.

### Example 6: Enriching 5' tri-phosphorylated polIII RNA from total human RNA

Ribo-Zero™ (Epicenter, CA) was used to remove human ribosomal RNA from the preparation of UHR RNA.

Approximately 100 ng of Ribo-Zero depleted UHR RNA was mixed with m7G capped Fluc transcript (0.07 ng) and 5' triphosphate Cluc transcript (0.12ng). This was incubated in a 70 µl reaction volume with 1 x VCE buffer, 0.5 mM 3'desthiobiotin-GTP and 60 units of VCE for 2 hours at 37°C.

The resulting reaction was applied to a MEGAclear spin column and eluted with 50 µl of water. A 20 µl aliquot was saved as unenriched sample. 30 µl of the desthiobiotin capped RNA was added to 95 µl of 10 mM Tris HCl pH 7.5, 50 mM NaCl buffer containing 2 µl of murine RNAse inhibitor and was adsorbed to 125 µl of hydrophilic streptavidin beads that had been previously washed with 3 times with 10 mM Tris HCl pH 7.5, 500 mM NaCl buffer followed by washing in 10 mM Tris HCl pH 7.5, 50 mM NaCl buffer. The beads were incubated at room temperature for 20 minutes with occasional mixing. The beads were then washed 2 times with 500 µl of 10 mM Tris-HCl pH 7.5, 1 mM EDTA, 500 mM NaCl and then 2 times with 500 µl of 10 mM Tris-HCl pH 7.5, 50 mM NaCl, 1 mM EDTA. All four washes contained 2 µl murine RNAase inhibitor. The beads were then suspended in 100 µl of 10 mM Tris-HCl pH 7.5, 1 mM EDTA, 50 mM NaCl containing 1 mM biotin, 2 µl murine RNAase inhibitor and incubated at room temperature for 20 minutes with occasional mixing. The beads were collected on the side of the tube with a magnet and the 100 µl supernatant was collected. The resulting biotin eluted RNA product and unenriched aliquot were then concentrated by use of "RNA Clean and Concentrator" in 10 µl of water. The three RNA samples were prepared for sequencing by using the NEBNext Ultra Directional RNA Library Prep Kit for Illumina as described by the manufacturer and sequenced on the Illumina MiSeq.

Sequencing reads were quality filtered and the adaptors were trimmed. The reads were mapped to the human genome (hg19) using STAR [STAR: ultrafast universal RNA-seq aligner. Dobin]. The mapped reads were overlapped with annotated features from gencode v17 (PUBMED: 22955987) and the repeat features from UCSC (hg19). We defined human polymerase III (pol III) transcripts annotated as 7SK and 7SL where pol III has a 5' triphosphate, small nuclear RNA, Alu repeats and tRNAs. We defined polymerase II (pol II) transcripts as transcripts annotated with the term "protein coding" and these transcripts are characterized by being capped.

As shown in FIG. 6, the human RNA polymerase III transcripts were enriched about 5-10 fold and the human RNA polymerase II transcripts were depleted.

### Example 7: Comparison of enzymatic release of RNA with competitive elution using biotin of RNA bound to streptavidin beads.

The RNA that had been prepared and bound to streptavidin beads as described in Example 1 and 3 was treated with (i) either *Vaccinia* decapping enzyme D9 or *Vaccinia* decapping enzyme D10 (Parrish, et al., J. Virol., 81(23):12973-8 (2007)), or (ii) by addition of biotin to the immobilized RNA (as in Example 3). It was shown that both decapping enzymes successfully removed bound capped RNA from the beads. Thus, like elution of biotin-capped RNA using biotin, decapping enzymes are equally suitable for releasing captured capped RNA.

### Example 8: Analysis of Transcription start sites (TSS) by RNA-SEQ of enriched RNA from a prokaryote in a method with less than single base resolution

RNA was fragmented before the enrichment step following the protocol described below.

For the sample: 6 ug of total *E. coli* RNA and for controls: 7 ng of Fluc RNA and 24 ng of Cluc RNA; were incubated in a 70 µl reaction volume with 1 x VCE buffer, 0.5 mM 3'desthiobiotin-GTP and 60 units of VCE for 2 hours at 37°C. The resulting reaction was applied to a MEGAclear spin column and eluted with 100 µl water.

The resulting RNA was reduced in size to about 200 nucleotides by incubation at 94 degrees for ten minutes in the presence of 3.3x NEB First Strand Synthesis Buffer (New England Biolabs, Ipswich, MA).

A 30 µl aliquot was saved as unenriched sample. 35 µl of the desthiobiotin capped RNA was added to 70 µl of 10 mM Tris HCl pH 7.5, 50 mM NaCl buffer containing 2 µl of murine RNAse inhibitor. This mixture was adsorbed to 125 µl of hydrophilic streptavidin beads that had been previously washed with 3 times with 10 mM Tris HCl pH 7.5, 500 mM NaCl buffer followed by washing in 10 mM Tris HCl pH 7.5, 50 mM NaCl buffer. The beads were incubated at room temperature for 20 minutes with occasional mixing. The beads were then washed 2 times with 500 µl of 10 mM Tris-HCl pH 7.5, 1 mM EDTA, 500 mM NaCl for and then 2 times with 500 µl of 10 mM Tris-HCl pH 7.5, 50 mM NaCl, 1 mM EDTA all four washes contained 2 µl murine RNAase inhibitor. The beads were then suspended in 100 µl of 10 mM Tris-HCl pH 7.5, 1 mM EDTA, 50 mM NaCl containing 1 mM biotin, 2 µl murine RNAase inhibitor and incubated at room temperature for 20 minutes with occasional mixing. The beads were collected on the side of the tube with a magnet and the 100 µl supernatant was collected. The resulting biotin eluted RNA product and unenriched sample were then concentrated by use of "RNA Clean and Concentrator" in 10 µl of water. The three RNA samples were prepared for sequencing by using the NEBNext Ultra Directional RNA Library Prep Kit for Illumina as described by the manufacturer and sequenced on the Illumina MiSeq.

Sequencing reads were quality filtered and the adaptors were trimmed. The reads were mapped to *E.coli* (U00096.2) genome and CLUC and FLUC transcript sequences using BWA [PMID:19451168].

The results in FIG. 7 shows an example of 5kb *E. coli* genomic region (panel A) with the annotated genes (Panel D) and annotated TSS (Panel E) (see Kim, et al., PLoS genetics, 8(8), e1002867 (2012)). The un-enriched sample when sequenced provided a large number of reads (Panel B). The reads distribute more or less uniformly across the full lengths of the annotated genes. In contrast, the sample in Panel C was enriched and the number of sequencing reads was reduced with discrete peaks (although not single base resolution) at TSS at the beginning of the annotated genes. The maximum signal of reads for each gene correlated precisely with the annotated TSS (Panel E) with an additional peak at the gadW gene indicative of a novel TSS. This demonstrates the power of this method to identify TSS that were previously unknown.

The data in Table 3 confirms efficient depletion of ribosomal RNA from more than 95% of the reads in the fraction before enrichment to only about 20% in the fraction after enrichment.

**Table 3: Demonstrated Enrichment**

| Reads | Before Enrichment | After Enrichment |
|---|---|---|
| Non-ribosomal RNA | 508658 | 3404912 |
| Ribosomal RNA | 13620039 | 863987 |

### EXAMPLE 9: Analysis of Transcription start sites (TSS) by small RNA library preparation of enriched RNA from a prokaryote in a method with single base resolution

Total RNA was obtained from lysed *E.coli* and enriched by capping with VCE and desthiobiotinylated nucleotide and binding to streptavidin beads. The beads were washed before elution with biotin.

7.5 µg of total *E. coli* RNA was incubated at 70 degrees for 2 minutes in a 1 mM Tris-HCl pH 8.0, 0.1 mM NaCl the buffer was then adjusted to contain 50 mM Tris-HCl pH 8.0, 5 mM KCl, 1 mM MgCl2, 1 mM DTT, 0.1 mM SAM, 0.5 mM 3'desthiobiotin-GTP and 50 units of VCE for 30 minutes at 37°C. The RNA was then isolated by use of "RNA Clean and Concentrator" and eluted by 100 µl of 1mM Tris-HCl pH 8.0, 0.1 mM EDTA.

The RNA was fragmented by adding 2.5 µl of 10X T4 Polynucleotide Kinase Buffer (absent of any ATP) to the 100 µl solution and was heated at 94°C for 5 minutes. The RNA was collected by exposure to 1.8 volumes of AMPure® XP beads (Beckman Coulter, Indianapolis, IN) with an additional 1.5 volumes of 100% ethanol. The beads were washed with 80% ethanol two times and then dried for five minutes and eluted with 100 µl of 1 mM Tris-HCI ph 7.5, 0.1 mM EDTA. The 3' ends of RNA were dephosphorylated by incubating 75 µl of the RNA solution in 1X T4 polynucleotide kinase buffer with 40 units of T4 Polynucleotide Kinase (previously dialyzed in ATP-free kinase storage buffer) in a total volume of 82 µl for 15 minutes at 37°C.

The 75 µl of kinase treated RNA was divided into a 25 µl and 50 µl volume.

The 25 µl volume of the kinase treated RNA was purified by AMPure XP beads as described above and eluted in 30 µl of 1 mM Tris-HCl ph 7.5, 0.1 mM EDTA. To the 50 volume of kinase treated RNA was added 30 µl of prewashed streptavidin beads. The beads were then washed 2 times with 500 µl of 10 mM Tris-HCl pH 7.5, 1 mM EDTA, 500 mM NaCl for and then 2 times with 500 µl of 10 mM Tris-HCl pH 7.5, 1 mM EDTA. The beads were collected on the side of the tube with a magnet and 30 µl supernatant was collected. This 30 µl was subjected to AMPure XP bead/ethanol cleanup as above and eluted in 60 µl of 1 mM Tris-HCl, 0.1 mM EDTA. The 60 µl solution was split into two 30 µl samples. One of the two 30 µl samples was subjected to another round of streptavidin binding, washing and elution and AMPure XP bead cleanup as described above. The three samples (a) no streptavidin enrichment, (b) 1 round of streptavidin enrichment and (c) 2 rounds of streptavidin enrichment were all subjected to RppH decapping as described here: to the 30 µl of solution was added 3.3 µl of 10X ThermoPol® Buffer (New England Biolabs, Ipswich, MA) and 15 units of RppH (New England Biolabs, Ipswich, MA) and incubated for 60 minutes at 37°C. 0.5 µl of 0.5 M EDTA was added to each sample and then heated to 94°C for 2 minutes. The samples were then collected on AMPure XP beads as previously and eluted in 20 µl of 1 mM Tris-HCI, 0.1 mM EDTA.

The three RNA samples were prepared for sequencing by using the NEBNext Small RNA Library Prep Kit for Illumina as described by the manufacturer and sequenced on the Illumina MiSeq.

The results shown in FIG. 8 depict the relative amount of RNA found from ribosomal and intergenic and protein coding regions in the unenriched fraction versus the enriched fraction (two streptavidin rounds).

The results are shown in FIG. 9 for single base resolution of the TSS. Single base resolution is obtained (panel - bound fraction) that exactly corresponds to the TSS of genes shown in panel (TSS). Panel - total RNA fraction shows the number of reads obtained at each position along the genome fragment from non-enriched (total), fragmented, 5' modified, capped RNA.

Here we define the TSS by requiring a minimum of 20 reads initiating at the same nucleotide position and compare this read distribution to that obtained from un-enriched total RNA requiring an enrichment of a base 2 logarithm greater than 1.

### Example 10: Analysis of a metatranscriptome (e.g. microbiome)

With the recognition of the importance of the microbiome for human health, efforts have proceeded to obtain genome sequences for all the representative organisms in a microbiome to ascertain which organisms are present and how the population of microorginisms might vary in health and disease. A list of occurrences of microorganisms does not reveal the functional state of each organism. The level of transcription is not revealed with genomic DNA sequence. However transcriptional activity or gene expression level does reflect the active functional state of an organism. Another approach presented here is to analyze the entire RNA population of the microbiome after enrichment away from ribosomal and host RNA.

RNA from fecal matter is obtained by use of for example, PowerMicrobiome™ RNA Isolation Kit (Mo Bio, Carlsbad, CA). The obtained prokaryotic RNA is capped with a capping enzyme and a labeled modified nucleotide and the labeled RNA is reacted with coated beads that are capable of binding to the label on the capped RNA. All unbound RNA is washed away and the bound RNA is collected. This RNA is then sequenced using high throughput sequencing. The conditions optimized for a feces derived microbiome and a metatranscriptome may be optimized by first testing a synthetic microbiome which may contain a plurality of known organisms in a synthetic mixture (for example, 4 microorganism lysates). After high throughput sequencing, the sequences are mapped and quantified with respect to a database of genomes. These results characterize the relative expression of RNA transcripts in the microbiome of the host animal (for example, a mouse).

### Example 11: Sequencing of the 5' end of Nascent eukaryotic RNA

Total RNA is isolated from eukaryotic cells using trizol and precipitated with ethanol. The lower molecular weight fraction of total RNA (below 100 nucleotides) is obtained by AMPure XP bead differential size selection. Small RNA is enriched by first capping with 3' desthiobiotin GTP according to the methods described in Example 9. Half of the RNA is used as control, the remaining is adsorbed to hydrophilic Streptavidin beads and washed and then eluted with biotin. The RNA is decapped with RppH. The RNA is prepared for sequencing using NEBNext Small RNA Library Prep Kit or subjected to template switching library preparation (SMART® Ultralow RNA Kit, Clontech, Mountain View, CA) and sequenced using MiSEQ. Prior to library preparation, the RNA can be released from streptavidin beads either in the presence of biotin or by decapping. Decapping or denaturation of the streptavidin would be preferred if the modified nucleotide used had a label for binding coated beads that was not readily elutable without decapping or denaturation of the streptavidin.

The resulting sequences will represent the position of the TSS of nascent RNA on the eukaryotic genome and the relative abundance of each nascent transcript.

### Example 12: Template switching to reveal 5' end of Nascent Eukaryotic RNA

Total RNA is isolated from eukaryotic cells using trizol and precipitated with ethanol. The lower molecular weight fraction of total RNA (below 100 nucleotides) is obtained by AMPure XP bead differential size selection. In one embodiment, small RNA is enriched by first capping with desthiobiotin GTP according to the methods described in Example 9. The RNA is adsorbed to hydrophilic Streptavidin beads and washed and then eluted with biotin. This labeled capped RNAs can be reverse transcribed using a template switching oligonucleotide (New England Biolabs) which enables introduction of a necessary priming sequence for the generation of DNA libraries for NextGen sequencing (see FIG. 12) with the expectation of single nucleotide resolution.

### Example 13: Cap Jumping to identify di- and tri-phosphorylated transcripts.

In this example total RNA is obtained from a bacterial culture and enriched by capping with VCE and a nucleotide modified at its 3' position with a linker attached to an oligonucleotide (see FIG. 2C and FIG. 2D). When the label of the capping nucleotide is composed of a oligonucleotide (FIG. 11), cap jumping (see for example, Efimov, et al., Nucleic Acids Res., 29(22):4751-4759 (2001)) can be used to introduce the necessary priming sequence for the generation of DNA libraries for NextGen sequencing. In place of enrichment by affinity binding to a solid support, an oligonucleotide is attached through a capping reaction for direct use in amplification. Only artificially capped RNA is successfully reverse transcribed resulting in an adapter sequence for amplification. As shown in FIG. 11, it may be possible to attach an oligonucleotide label by the 5' end or the 3' end to the linker on the modified nucleotide. In one case, reverse transcription can jump to oligonucleotides of modified CAP with 3' to 5' polarity or vice versa. Using this approach, enrichment might be achieved by selection of amplicons as only those RNAs with tri- or di-phosphorylated 5' ends will be amenable to amplification.

### Example 14: Association of sequence specific markers (SSM) with a phenotype

In this example, two cohorts of several hundred to several thousand humans are selected. One cohort is composed of patients with a disease condition, the other cohort is composed of healthy individuals or patients with another disease condition. Disease conditions can be for example, Crohn's disease, celiac disease, infections with or without resistance to a given antibiotics, diabetes, or pneumonia. For each individual, samples from the relevant body part can be taken for example an intestinal mucosal biopsy can be obtain using the intestinal mucosal brush methodology. From the biological sample, total RNA can be extracted using for example the PowerMicrobiome RNA Isolation Kit and 5' triphosphate RNA fragments can be isolated using for example the procedure described in Examples 9. For each initial sample, sequencing reads are adaptor trimmed and shortened to the desired length of the SSM (for example 30 bp) and processed into a data structure composed of a hash table with the key being the reads and the value being the incrementation of a particular key.

For each cohort, a matrix can be derived where rows correspond to SSMs and columns correspond to individual sample. Using established linkage statistical methods, a set of significant SSMs associated to a given disease can be derived from comparing the two matrices. See for example FIG. 10 as a prophetic example where associated SSMs are depicted by a heat map of a cohort of 4 treated subjects and a cohort of 4 controls subjects. Those associated SSMs can be used for diagnostic purpose or can be used to find the causative genes or microbial species for, for example, drug development.

### Example 15: Alternative method for obtaining a Signature

An alternative method for generating a signature may be obtained after NEBNext Ultra Directional RNA Library Preparation and sequencing. The RNA can be directly used for library preparation without decapping. Sequencing reads are assembled using existing algorithm for example trinity [Trinity: reconstructing a full-length transcriptome without a genome from RNA-Seq data] to assembled transcripts. Each assembled transcript would correspond to a SSMs and the collection of transcripts represent the signature. The quantitative value of a given SSM is the number of reads mapping to the SSM.

### Example 16: Transcriptome analysis of a prokaryote by obtaining and characterizing non-ribosomal prokaryotic RNA

Prokaryotic non ribosomal RNA was enriched from prokaryotic total RNA and characterized by Nextgen sequencing

15 µg of total *E. coli* RNA was incubated at 65 degrees for 5 minutes in water. The RNA solution was adjusted to contain 50 mM Tris-HCl pH 8.0, 5 mM KCl, 1 mM MgCl2, 1 mM DTT, 0.1 mM SAM, 200 units RNAase inhibitor, 0.5 mM 3'desthiobiotin-GTP and 250 units of VCE for 35 minutes at 37°C. The RNA solution was aliquoted into two equal volumes and isolated by use of "RNA Clean and Concentrator" and each column was eluted by 50 µl water and then combined to 100 µl. The NaCl concentration was brought to 0.25 M by adding 100 µl of 0.5 M NaCl.

133 µl of the RNA solution was added to prewashed streptavidin beads representing 100 µl original bead volume. These beads were washed and eluted as described in Example 6 (Enriched fraction 1). Likewise the remaining 66 µl of RNA solution was added to 50 µl of beads. These beads were washed and eluted as described in example 6 except that the 10 mM Tris-HCL, 1 mM EDTA, 50 mM NaCl wash solution was substituted with 10 mM Tris-HCL, 1 mM EDTA. And the 0.5 M NaCl solution was substituted with 10 mM Tris-HCL, 1 mM EDTA, 2 M NaCl (Enriched fraction 2).

The samples were then individually collected with 1.8 volume of AMPure XP beads and eluted in 50 µl of 10 mM Tris-HCl, 1 mM EDTA. The two samples were then subjected to another round of streptavidin bead enrichment as described above. The samples were then collected with 1.8 volume of AMPure XP beads and eluted in 50 µl of 10 mM Tris-HCl, 1 mM EDTA. These two samples (Enriched fraction 1 and Enriched fraction 2) as well as the starting total RNA were used to make NEBNext Ultra Directional RNA libraries and sequenced on an Illumina MiSeq.

FIG. 5 depicts the percentage of reads mapping to ribosomal genes, protein coding genes or intergenic regions of the *E.coli* (U00096.2) genome for total RNA and enriched RNA fractions. Reads were mapped to the *E.coli* genome (U00096.2). As can be seen in FIG. 5 the enriched RNA fraction is depleted of rRNA in both bead washing procedures (Enriched fraction 1 and 2). The *E. coli* non-ribosomal RNA was enriched to similar extents with both bead washing procedures.

## Claims

1. A method of forming a 5' capped labelled RNA, comprising:
combining a preparation comprising uncapped RNA having a 5' terminal diphosphate or triphosphate with a capping enzyme and a compound represented by Formula (I): 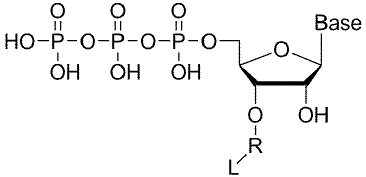
or a salt thereof, wherein:
Base is a purine or a pyrimidine,
R is a linker, and
L is a label selected from the group consisting of an affinity label, a detection
label, a chemoselective group, an oligonucleotide, and a combination thereof; so as to convert the uncapped RNA into 5' labelled capped RNA.

2. A method according to Claim 1, wherein the uncapped RNA is nascent eukaryotic mRNA, mature uncapped eukaryotic mRNA, prokaryotic mRNA, or prokaryotic small RNAs.

3. The method according to Claim 1, further comprising enriching the labelled capped RNA by:
(a) immobilizing the 5' capped labelled RNA on an affinity substrate; or
(b) amplifying the 5' capped labelled RNA, wherein the label comprises an oligonucleotide.

4. A method according to Claim 1, wherein the capped RNA has a binding affinity to a matrix, and wherein the method further comprises selective binding of the 5' capped labelled RNA to the matrix.

5. A method according to Claim 4, wherein the label L is desthiobiotin or a derivative thereof, wherein the 5' capped labelled RNA binds reversibly to a matrix.

6. A method according to Claim 5, wherein the method further comprises the step of eluting the target RNA from the matrix using biotin.

7. A method according to any of Claims 4-6, wherein the RNA is:
a prokaryotic transcriptome; or a meta-transcriptome, such as wherein the meta-transcriptome is from a microbiome or from a eukaryotic tissue sample; or
nascent eukaryotic RNA and/or eukaryotic mRNA;
or wherein the RNA comprises transcription start sites (TSS).

8. The method according to any of Claims 4-7, further comprising adding adapters to the decapped ends of eluted RNA for reverse transcribing to DNA and amplifying the DNA prior to sequencing.

9. The method according to any of Claims 4-8, further comprising quantifying the RNA by quantifying sequencing reads, or by quantifying 5' transcriptional start sites (TTS) to obtain sequence specific markers for the RNA population, and optionally correlating the sequence specific markers with a phenotype of a eukaryotic host or a complex mixed population of microbes.

10. The method according to any preceding claim, wherein the RNA in the preparation comprises naturally capped RNA and prior to capping in the presence of the compound, the method further comprises: decapping the naturally capped RNA with a decapping enzyme wherein the uncapped RNA has a 5' terminal diphosphate or triphosphate.

11. A method for enriching for prokaryotic non-ribosomal RNA in a mixture comprising eukaryotic and prokaryotic RNA, comprising:
(a) combining a mixture of RNA comprising eukaryotic and prokaryotic RNA with a compound represented by Formula (I): 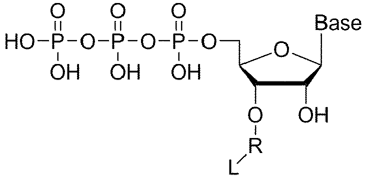 or a salt thereof, wherein:
Base is a purine or a pyrimidine,
R is a linker, and
L is a label selected from the group consisting of an affinity label, a detection
label, a chemoselective group, an oligonucleotide, and a combination thereof;
in the presence of a capping enzyme, so as to form 5' labelled capped prokaryotic non-ribosomal RNA;
(b) immobilizing the 5' capped labelled RNA; and
(c) removing unreacted RNA.

12. The method according to Claim 11, further comprising:
eluting the immobilized 5' capped labelled RNA; and/ or
hybridizing the 5' capped labelled RNA to a probe for identifying the immobilized 5' capped labeled RNA; wherein the step of hybridizing occurs when the RNA is immobilized or after the RNA is eluted from a matrix.

13. The method according to Claim 11 or 12, wherein:
the RNA sequences or the identified RNAs are compiled into a transcriptome; or
the RNA sequences or the identified RNAs are sequence specific markers; such as sequence specific markers for a transcriptome or metatranscriptome, or wherein the sequence specific markers form a signature profile; or
the RNA sequences comprise or correspond to transcriptional start sites (TSS).

14. A method for determining transcriptional start sites (TSS) of RNA, comprising:
(a) obtaining total RNA from prokaryotic cells, eukaryotic cells or a mixture of both eukaryotic and prokaryotic cells;
(b) capping uncapped or decapped RNA having a 5' diphosphorylated or 5' tri-phosphorylated end from the total RNA with a compound represented by Formula (I): 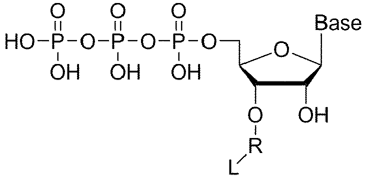 or a salt thereof, wherein:
Base is a purine or a pyrimidine,
R is a linker, and
L is a label selected from the group consisting of an affinity label, a detection
label, a chemoselective group, an oligonucleotide, and a combination thereof;
in the presence of a capping enzyme, thereby forming 5' capped labelled RNA; and
(c) sequencing the 5' capped labelled RNA or hybridizing the 5' capped RNA to a probe so as to determine the transcription start sites (TSS) of the RNA.

15. A method according to Claim 14, wherein the uncapped RNA is nascent eukaryotic RNA, mature uncapped eukaryotic mRNA, prokaryotic mRNA, or prokaryotic small RNAs.

16. A method according to any of Claims 1 to 15, further comprising: fragmenting the RNA before or after formation of the 5' labelled capped RNA.

17. A method of selectively labelling uncapped RNA having a 5' tri-phosphate or a 5' di-phosphate in a population of RNAs, comprising:
combining the population of RNAs with a compound represented by Formula (I): 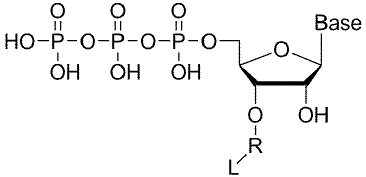
or a salt thereof, wherein:
Base is a purine or a pyrimidine,
R is a linker, and
L is a label selected from the group consisting of an affinity label, a detection
label, a chemoselective group, an oligonucleotide, and a combination thereof;
so as to convert the uncapped RNA into selectively 5' labelled capped RNA;
wherein the RNA is fragmented into oligonucleotides before or after selective labelling.

18. A method according to Claim 17, further comprising hybridizing the labelled oligonucleotides with a probe to obtain 5' sequence specific markers.

19. The method according to Claim 17 or 18, further comprising correlating the 5' signature sequence profile of a transcriptome with a phenotype of a eukaryotic or prokaryotic cell or cells.

20. The method according to any preceding claim, wherein the method comprises sequencing the 5' capped labelled RNA.

21. The method of any preceding claim, wherein the detection label is a fluorescent label; or wherein the label is an affinity label selected from the group consisting of a biotin moiety, desthiobiotin, avidin, streptavidin, protein A, maltose-binding protein, poly-histidine, HA-tag, c-myc tag, FLAG-tag, SNAP-tag, S-tag and glutathione-S-transferase (GST); preferably a biotin moiety or desthiobiotin.

22. The method of any preceding claim, wherein the base is a purine; wherein the purine is selected from a guanosine, inosine or an analog thereof.

23. The method of any preceding claim, wherein the compound is 3'-O-(2-aminoethylcarbamoyl) (EDA)-biotin guanosine tri-phosphate (GTP) or 3'-desthiobiotin-tetraethylene glycol (TEG)-GTP.

24. The method according to any preceding claim, wherein the capping step is performed using a capping enzyme selected from a Vaccinia capping enzyme (VCE), a Bluetongue Virus capping enzyme, a Chlorella Virus capping enzyme, or a *S*. *cerevisiae* capping enzyme.

25. A preparation or kit comprising a capping enzyme and a compound represented by Formula (I): 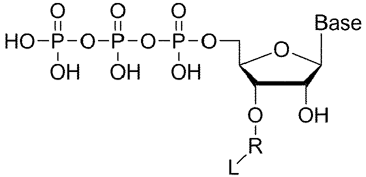 or a salt thereof, wherein:
Base is a purine or a pyrimidine,
R is a linker, wherein the linker is a straight or branched chain alkyene group with 1 to 300 carbon atoms, and
L is a label selected from the group consisting of an affinity label, a detection
label, a chemoselective group, an oligonucleotide, and a combination thereof.

26. The preparation or kit of claim 25, wherein:
the detection label is a fluorescent label; or wherein the label is an affinity label selected from the group consisting of a biotin moiety, desthiobiotin, avidin, streptavidin, protein A, maltose-binding protein, poly-histidine, HA-tag, c-myc tag, FLAG-tag, SNAP-tag, S-tag and glutathione-S-transferase (GST); preferably a biotin moiety or desthiobiotin; and/or
the base is a purine; wherein the purine is selected from a guanosine, inosine or an analog thereof.

27. The preparation or kit of Claim 26, wherein the compound is 3'-O-(2-aminoethylcarbamoyl) (EDA)-biotin guanosine tri-phosphate (GTP) or 3'-desthiobiotin-tetraethylene glycol (TEG)-GTP.

28. The preparation or kit of any of claims 25-27, wherein the capping enzyme is a Vaccinia capping enzyme (VCE), a Bluetongue Virus capping enzyme, a Chlorella Virus capping enzyme, or a *S*. *cerevisiae* capping enzyme.

29. The kit of any of claims 25-28, wherein the kit further comprises an affinity matrix.

## Patentansprüche

1. Ein Verfahren zum Bilden einer mit 5'-Cap versehenen markierten RNA, das Folgendes beinhaltet:
Kombinieren einer Zubereitung, die nicht mit einer Cap versehene RNA mit einem 5'-endständigen Diphosphat oder Triphosphat beinhaltet, mit einem Capping-Enzym und einer Verbindung, dargestellt durch Formel (I): 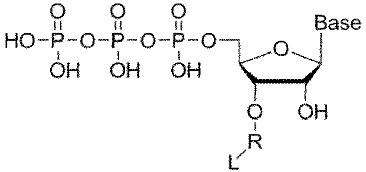
oder eines Salzes davon, wobei:
Base ein Purin oder ein Pyrimidin ist,
R ein Linker ist und
L eine Markierung ist, ausgewählt aus der Gruppe, bestehend aus einer Affinitätsmarkierung, einer Nachweismarkierung, einer chemoselektiven Gruppe, einem Oligonukleotid und einer Kombination davon;
um somit die nicht mit einer Cap versehene RNA in 5'-markierte mit einer Cap versehene RNA umzuwandeln.

2. Verfahren nach Anspruch 1, wobei die nicht mit einer Cap versehene RNA naszierende eukaryotische mRNA, reife eukaryotische nicht mit einer Cap versehene mRNA, prokaryotische mRNA oder prokaryotische kleine RNAs ist.

3. Verfahren nach Anspruch 1, das ferner das Anreichern der markierten mit einer Cap versehene RNA durch Folgendes beinhaltet:
(a) Immobilisieren der mit 5'-Cap versehenen markierten RNA auf einem Affinitätssubstrat; oder
(b) Amplifizieren der mit 5'-Cap versehenen markierten RNA, wobei die Markierung ein Oligonukleotid beinhaltet.

4. Verfahren nach Anspruch 1, wobei die mit einer Cap versehene RNA eine Bindungsaffinität zu einer Matrix aufweist und wobei das Verfahren ferner die selektive Bindung der mit 5'-Cap versehenen markierten RNA an der Matrix beinhaltet.

5. Verfahren nach Anspruch 4, wobei die Markierung L Desthiobiotin oder ein Derivat davon ist, wobei die mit 5'-Cap versehene markierte RNA reversibel an eine Matrix bindet.

6. Verfahren nach Anspruch 5, wobei das Verfahren ferner den Schritt des Eluierens der Ziel-RNA aus der Matrix unter Verwendung von Biotin beinhaltet.

7. Verfahren nach einem der Ansprüche 4-6, wobei die RNA Folgendes ist:
ein prokaryotisches Transkriptom; oder ein Meta-Transkriptom, wobei zum Beispiel das Meta-Transkriptom von einem Mikrobiom oder von einer eukaryotischen Gewebeprobe stammt; oder
naszierende eukaryotische RNA und/oder eukaryotische mRNA;
oder wobei die RNA Transkriptionsstartstellen(TSS) beinhaltet.

8. Verfahren nach einem der Ansprüche 4-7, das ferner das Hinzufügen von Adaptern zu den nicht mehr mit einer Cap versehenen Enden der eluierten RNA zum Umkehr-Transkribieren zu DNA und Amplifizieren der DNA vor der Sequenzierung beinhaltet.

9. Verfahren nach einem der Ansprüche 4-8, das ferner Folgendes beinhaltet: das Quantifizieren der RNA durch Quantifizieren von Sequenzierungs-Reads oder durch Quantifizieren von 5'-Transkriptionsstartstellen (TTS), um sequenzspezifische Marker für die RNA-Population zu erhalten, und optional das Korrelieren der sequenzspezifischen Marker mit einem Phänotyp eines eukaryotischen Wirts oder einer komplexen gemischten Population von Mikroben.

10. Verfahren nach einem der vorhergehenden Ansprüche, wobei die RNA in der Zubereitung natürlich mit einer Cap versehene RNA beinhaltet und wobei das Verfahren vor dem Capping in Gegenwart der Verbindung ferner Folgendes beinhaltet: Decapping der natürlich mit einer Cap versehenen RNA mit einem Decapping-Enzym, wobei die nicht mit einer Cap versehene RNA ein 5'-endständiges Diphosphat oder Triphosphat aufweist.

11. Verfahren zum Anreichern für prokaryotische nichtribosomale RNA in einer Mischung, die eukaryotische und prokaryotische RNA beinhaltet, das Folgendes beinhaltet:
(a) Kombinieren einer Mischung von RNA, die eukaryotische und prokaryotische RNA beinhaltet, mit einer Verbindung, dargestellt durch Formel (I): 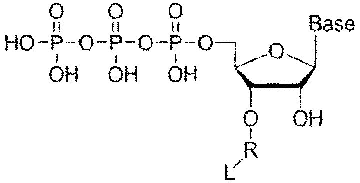 oder einem Salz davon, wobei:
Base ein Purin oder ein Pyrimidin ist,
R ein Linker ist und
L eine Markierung ist, ausgewählt aus der Gruppe, bestehend aus einer Affinitätsmarkierung, einer Nachweismarkierung, einer chemoselektiven Gruppe, einem Oligonukleotid und einer Kombination davon;
in Gegenwart eines Capping-Enzyms, um somit eine 5'-markierte prokaryotische nichtribosomale mit einer Cap versehene RNA zu bilden;
(b) Immobilisieren der mit 5'-Cap versehenen markierten RNA; und
(c) Entfernen von nicht umgesetzer RNA.

12. Verfahren nach Anspruch 11, das ferner Folgendes beinhaltet:
Eluieren der immobilisierten mit 5'-Cap versehenen markierten RNA; und/oder
Hybridisieren der mit 5'-Cap versehenen markierten RNA an eine Sonde zum Identifizieren der immobilisierten mit 5'-Cap versehenen markierten RNA; wobei der Schritt des Hybridisierens eintritt, wenn die RNA immobilisiert ist oder nach dem Eluieren der RNA aus einer Matrix.

13. Verfahren nach Anspruch 11 oder 12, wobei:
die RNA-Sequenzen oder die identifizierten RNAs in einem Transkriptom zusammengestellt werden; oder
die RNA-Sequenzen oder die identifizierten RNAs sequenzspezifische Marker sind; wie etwa sequenzspezifische Marker für ein Transkriptom oder Meta-Transkriptom, oder wobei die sequenzspezifischen Marker ein Signaturprofil bilden; oder
die RNA-Sequenzen Transkriptionsstartstellen (TSS) beinhalten oder diesen entsprechen.

14. Verfahren zum Bestimmen von Transkriptionsstartstellen (TSS) von RNA, das Folgendes beinhaltet:
(a) Erhalten von Gesamt-RNA von prokaryotischen Zellen, eukaryotischen Zellen oder einer Mischung sowohl von eukaryotischen als auch prokaryotischen Zellen;
(b) Capping von nicht mit einer Cap versehener oder nicht mehr mit einer Cap versehener RNA mit einem 5'-diphosphorylierten oder 5'-triphosphorylierten Ende aus der Gesamt-RNA mit einer Verbindung, dargestellt durch Formel (I) : 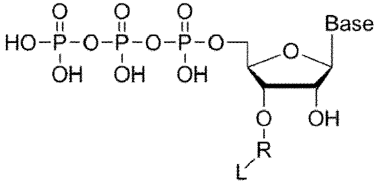 oder einem Salz davon, wobei:
Base ein Purin oder ein Pyrimidin ist,
R ein Linker ist und
L eine Markierung ist, ausgewählt aus der Gruppe, bestehend aus einer Affinitätsmarkierung, einer Nachweismarkierung, einer chemoselektiven Gruppe, einem Oligonukleotid und einer Kombination davon;
in Gegenwart eines Capping-Enzyms, wodurch mit 5'-Cap versehene markierte RNA gebildet wird; und
(c) Sequenzieren der mit 5'-Cap versehenen markierten RNA oder Hybridisieren der mit 5'-Cap versehenen RNA an eine Sonde, um somit die Transkriptionsstartstellen (TSS) der RNA zu bestimmen.

15. Verfahren nach Anspruch 14, wobei die nicht mit einer Cap versehene RNA eine naszierende eukaryotische RNA, reife eukaryotisch nicht mit einer Cap versehene mRNA, prokaryotische mRNA oder prokaryotische kleine RNAs ist.

16. Verfahren nach einem der Ansprüche 1 bis 15, das ferner Folgendes beinhaltet: Fragmentieren der RNA vor oder nach der Bildung der 5'-markierten mit einer Cap versehenen RNA.

17. Verfahren zum selektiven Markieren von nicht mit einer Cap versehenet RNA mit einem 5'-Triphosphat oder einem 5'-Diphosphat in einer Population von RNAs, das Folgendes beinhaltet:
Kombinieren der Population von RNAs mit einer Verbindung, dargestellt durch Formel (I): 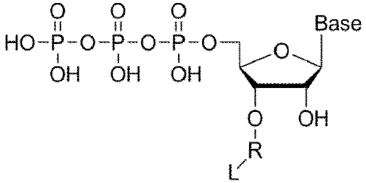
oder einem Salz davon, wobei:
Base ein Purin oder ein Pyrimidin ist,
R ein Linker ist und
L eine Markierung ist, ausgewählt aus der Gruppe, bestehend aus einer Affinitätsmarkierung, einer Nachweismarkierung, einer chemoselektiven Gruppe, einem Oligonukleotid und einer Kombination davon;
um somit die nicht mit einer Cap versehene RNA in selektiv 5'-markierte mit einer Cap versehene RNA umzuwandeln;
wobei die RNA vor oder nach dem selektiven Markieren in Oligonukleotide fragmentiert wird.

18. Verfahren nach Anspruch 17, das ferner das Hybridisieren der markierten Oligonukleotide mit einer Sonde beinhaltet, um 5'-sequenzspezifische Marker zu erhalten.

19. Verfahren nach Anspruch 17 oder 18, das ferner das Korrelieren des 5'-Signatursequenzprofils eines Transkriptoms mit einem Phänotyp einer eukaryotischen oder prokaryotischen Zelle oder Zellen beinhaltet.

20. Verfahren nach einem der vorhergehenden Ansprüche, wobei das Verfahren das Sequenzieren der mit 5'-Cap versehenen markierten RNA beinhaltet.

21. Verfahren nach einem vorhergehenden Anspruch, wobei die Nachweismarkierung eine fluoreszierende Markierung ist; oder wobei die Markierung eine Affinitätsmarkierung ist, ausgewählt aus der Gruppe, bestehend aus einem Biotinteil, Desthiobiotin, Avidin, Streptavidin, Protein A, Maltosebindungsprotein, Polyhistidin, HA-Tag, c-myc-Tag, FLAG-Tag, SNAP-Tag, S-Tag und Glutathion-S-transferase (GST); bevorzugt ein Biotinteil oder Desthiobiotin.

22. Verfahren nach einem vorhergehenden Anspruch, wobei die Base ein Purin ist; wobei das Purin aus einem Guanosin, Inosin oder einem Analogon davon ausgewählt ist.

23. Verfahren nach einem vorhergehenden Anspruch, wobei die Verbindung 3'-O-(2-Aminoethylcarbamoyl)(EDA)-Biotinguanosintriphosphat (GTP) oder 3'-Desthiobiotintetraethylenglykol(TEG)-GTP ist.

24. Verfahren nach einem der vorhergehenden Ansprüche, wobei der Capping-Schritt unter Verwendung eines Capping-Enzyms, ausgewählt aus einem Vaccinia-Capping-Enzym (VCE), einem Blauzungenvirus-Capping-Enzym, einem Chlorellavirus-Capping-Enzym oder einem S.*-cerevisiae-*Capping-Enzym, durchgeführt wird.

25. Eine Zubereitung oder ein Kit, die oder das Folgendes beinhaltet: ein Capping-Enzym und eine Verbindung, dargestellt durch Formel (I): 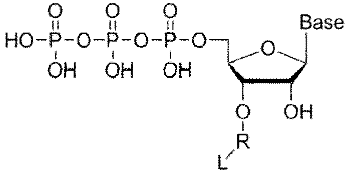 oder einem Salz davon, wobei:
Base ein Purin oder ein Pyrimidin ist,
R ein Linker ist, wobei der Linker eine geradkettige oder verzweigtkettige Alkyengruppe mit 1 bis 300 Kohlenstoffatomen ist und
L eine Markierung ist, ausgewählt aus der Gruppe, bestehend aus einer Affinitätsmarkierung, einer Nachweismarkierung, einer chemoselektiven Gruppe, einem Oligonukleotid und einer Kombination davon.

26. Zubereitung oder Kit nach Anspruch 25, wobei:
die Nachweismarkierung eine fluoreszierende Markierung ist; oder wobei die Markierung eine Affinitätsmarkierung ist, ausgewählt aus der Gruppe, bestehend aus einem Biotinteil, Desthiobiotin, Avidin, Streptavidin, Protein A, Maltosebindungsprotein, Polyhistidin, HA-Tag, c-myc-Tag, FLAG-Tag, SNAP-Tag, S-Tag und Glutathion-S-transferase (GST); bevorzugt ein Biotinteil oder Desthiobiotin; und/oder
die Base ein Purin ist; wobei das Purin aus einem Guanosin, Inosin oder einem Analogon davon ausgewählt ist.

27. Zubereitung eines Kits nach Anspruch 26, wobei die Verbindung 3'-O-(2-Aminoethylcarbamoyl)(EDA)-biotinguanosintriphosphat (GTP) oder 3'-Desthiobiotintetraethylenglykol(TEG)-GTP ist.

28. Zubereitung oder Kit nach einem der Ansprüche 25-27, wobei das Capping-Enzym ein Vaccinia-Capping-Enzym (VCE), ein Blauzungenvirus-Capping-Enzym, ein Chlorellavirus-Capping-Enzym oder ein S.*-cerevisiae-*Capping-Enzym ist.

29. Kit nach einem der Ansprüche 25-28, wobei das Kit ferner eine Affinitätsmatrix beinhaltet.

## Revendications

1. Procédé de formation d'un ARN marqué coiffé en 5', comprenant :
la combinaison d'une préparation comprenant un ARN non coiffé ayant un diphosphate ou triphosphate terminal en 5' avec une enzyme de coiffage et un composé représenté par la Formule (I) : 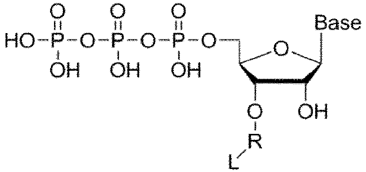
ou un sel de celui-ci, dans lequel :
la Base est une purine ou une pyrimidine,
R est un segment de liaison, et
L est un marqueur sélectionné parmi le groupe constitué par un marqueur d'affinité, un marqueur de détection, un groupe chimiosélectif, un oligonucléotide, et une combinaison de ceux-ci ;
de manière à convertir l'ARN non coiffé en ARN coiffé marqué en 5'.

2. Procédé selon la revendication 1, dans lequel l'ARN non coiffé est un ARNm eucaryote naissant, un ARNm eucaryote non coiffé mature, un ARNm procaryote ou des petits ARN procaryotes.

3. Procédé selon la revendication 1, comprenant en outre l'enrichissement de l'ARN coiffé marqué par :
(a) immobilisation de l'ARN marqué coiffé en 5' sur un substrat d'affinité ; ou
(b) amplification de l'ARN marqué coiffé en 5', dans lequel le marqueur comprend un oligonucléotide.

4. Procédé selon la revendication 1, dans lequel l'ARN coiffé a une affinité de liaison pour une matrice, et dans lequel le procédé comprend en outre la liaison sélective de l'ARN marqué coiffé en 5' à la matrice.

5. Procédé selon la revendication 4, dans lequel le marqueur L est la desthiobiotine ou un dérivé de celle-ci, dans lequel l'ARN marqué coiffé en 5' se lie de manière réversible à une matrice.

6. Procédé selon la revendication 5, dans lequel le procédé comprend en outre l'étape d'élution de l'ARN cible à partir de la matrice en utilisant la biotine.

7. Procédé selon l'une quelconque des revendications 4 à 6, dans lequel l'ARN est :
un transcriptome procaryote ; ou un méta-transcriptome, tel que dans lequel le métatranscriptome est issu d'un microbiome ou d'un échantillon de tissu eucaryote ; ou
un ARN eucaryote naissant et/ou un ARNm eucaryote ;
ou dans lequel l'ARN comprend des sites d'initiation de la transcription (TSS).

8. Procédé selon l'une quelconque des revendications 4 à 7, comprenant en outre l'ajout d'adaptateurs aux extrémités décoiffées de l'ARN élué pour la transcription inverse en ADN et l'amplification de l'ADN avant le séquençage.

9. Procédé selon l'une quelconque des revendications 4 à 8, comprenant en outre la quantification de l'ARN en quantifiant des lectures de séquençage, ou en quantifiant des sites d'initiation de la transcription (TTS) en 5' pour obtenir des marqueurs spécifiques de séquences pour la population d'ARN, et facultativement en faisant correspondre les marqueurs spécifiques de séquences avec un phénotype d'un hôte eucaryote ou d'une population mixte complexe de microbes.

10. Procédé selon l'une quelconque des revendications précédentes, dans lequel l'ARN dans la préparation comprend de l'ARN coiffé naturellement et avant le coiffage en présence du composé, le procédé comprend en outre : le décoiffage de l'ARN coiffé naturellement avec une enzyme de décoiffage dans lequel l'ARN décoiffé a un diphosphate ou un triphosphate terminal en 5'.

11. Procédé d'enrichissement en ARN procaryote non ribosomal dans un mélange comprenant de l'ARN eucaryote et procaryote, comprenant :
(a) la combinaison d'un mélange d'ARN comprenant de l'ARN eucaryote et procaryote avec un composé représenté par la Formule (I) : 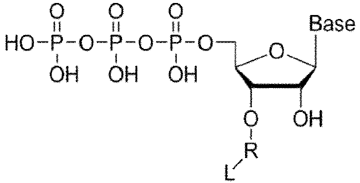 ou un sel de celui-ci, dans lequel :
la Base est une purine ou une pyrimidine,
R est un segment de liaison, et
L est un marqueur sélectionné parmi le groupe constitué par un marqueur d'affinité, un marqueur de détection, un groupe chimiosélectif, un oligonucléotide, et une combinaison de ceux-ci ;
en présence d'une enzyme de coiffage, de manière à former de l'ARN non ribosomal procaryote coiffé marqué en 5' ;
(b) l'immobilisation de l'ARN marqué coiffé en 5' ; et
(c) l'extraction de l'ARN n'ayant pas réagi.

12. Procédé selon la revendication 11, comprenant en outre :
l'élution de l'ARN marqué coiffé en 5' immobilisé ; et/ou
l'hybridation de l'ARN marqué coiffé en 5' à une sonde destinée à identifier l'ARN marqué coiffé en 5' immobilisé ; dans lequel l'étape d'hybridation a lieu quand l'ARN est immobilisé ou après l'élution de l'ARN à partir de la matrice.

13. Procédé selon la revendication 11 ou 12, dans lequel :
les séquences d'ARN ou les ARN identifiés sont rassemblés dans un transcriptome ; ou
les séquences d'ARN ou les ARN identifiés sont des marqueurs spécifiques de séquences ; tels que des marqueurs spécifiques de séquences pour un transcriptome ou un métatranscriptome, ou dans lequel les marqueurs spécifiques de séquences forment un profil de signature ; ou
les séquences d'ARN comprennent ou correspondent à des sites d'initiation de la transcription (TSS).

14. Procédé destiné à la détermination de sites d'initiation de la transcription (TSS) de l'ARN, comprenant :
(a) l'obtention d'ARN total à partir de cellules procaryotes, de cellules eucaryotes ou d'un mélange de cellules à la fois eucaryotes et procaryotes ;
(b) le coiffage d'ARN non coiffé ou décoiffé ayant une extrémité diphosphorylée en 5' ou triphosphorylée en 5' à partir de l'ARN total avec un composé représenté par la Formule (I) : 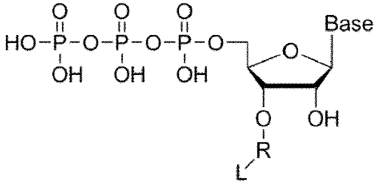 ou un sel de celui-ci, dans lequel :
la Base est une purine ou une pyrimidine,
R est un segment de liaison, et
L est un marqueur sélectionné parmi le groupe constitué par un marqueur d'affinité, un marqueur de détection, un marqueur chimiosélectif, un oligonucléotide, et une combinaison de ceux-ci ;
en présence d'une enzyme de coiffage, formant ainsi un ARN marqué coiffé en 5' ; et
(c) le séquençage de l'ARN marqué coiffé en 5' ou l'hybridation de l'ARN coiffé en 5' à une sonde afin de déterminer les sites d'initiation de la transcription (TSS) de l'ARN.

15. Procédé selon la revendication 14, dans lequel l'ARN non coiffé est un ARN eucaryote naissant, un ARNm eucaryote mature, un ARNm procaryote, ou des petits ARN procaryotes.

16. Procédé selon l'une quelconque des revendications 1 à 15, comprenant en outre : la fragmentation de l'ARN avant ou après la formation de l'ARN coiffé marqué en 5'.

17. Procédé de marquage sélectif d'ARN non coiffé ayant un triphosphate en 5' ou un diphosphate en 5' dans une population d'ARN, comprenant :
la combinaison de la population d'ARN avec un composé représenté par la Formule (I) : 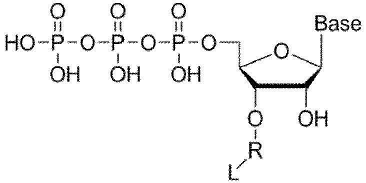
ou un sel de celui-ci, dans lequel :
la Base est une purine ou une pyrimidine,
R est un segment de liaison, et
L est un marqueur sélectionné parmi le groupe constitué par un marqueur d'affinité, un marqueur de détection, un groupe chimiosélectif, un oligonucléotide, et une combinaison de ceux-ci ;
de manière à convertir l'ARN non coiffé en ARN coiffé marqué en 5' sélectivement ;
dans lequel l'ARN est fragmenté en oligonucléotides avant ou après le marquage sélectif.

18. Procédé selon la revendication 17, comprenant en outre l'hybridation des oligonucléotides marqués avec une sonde pour obtenir des marqueurs spécifiques de séquences en 5'.

19. Procédé selon la revendication 17 ou 18, comprenant en outre la mise en correspondance du profil de séquence de signature en 5' d'un transcriptome avec un phénotype d'une cellule ou de cellules eucaryotes ou procaryotes.

20. Procédé selon l'une quelconque des revendications précédentes, dans lequel le procédé comprend le séquençage de l'ARN marqué coiffé en 5'.

21. Procédé selon l'une quelconque des revendications précédentes, dans lequel le marqueur de détection est un marqueur fluorescent ; ou dans lequel le marqueur est un marqueur d'affinité sélectionné parmi le groupe constitué par un groupement biotine, la desthiobiotine, l'avidine, la streptavidine, une protéine A, une protéine de liaison au maltose, une polyhistidine, une étiquette HA, une étiquette c-myc, une étiquette FLAG, une étiquette SNAP, une étiquette S et la glutathione-S-transférase (GST) ; préférablement un groupement biotine ou la desthiobiotine.

22. Procédé selon l'une quelconque des revendications précédentes, dans lequel la base est une purine ; dans lequel la purine est sélectionnée parmi une guanosine, une inosine ou un analogue de celle-ci.

23. Procédé selon l'une quelconque des revendications précédentes, dans lequel le composé est le 3'-O-(2-aminoéthylcarbamoyl) (EDA)-biotine guanosine tri-phosphate (GTP) ou le 3'-desthiobiotintétraéthylène glycol (TEG)-GTP.

24. Procédé selon l'une quelconque des revendications précédentes, dans lequel l'étape de coiffage est réalisée en utilisant une enzyme de coiffage sélectionnée parmi une enzyme de coiffage de la vaccine (VCE), une enzyme de coiffage du virus de la fièvre catarrhale, une enzyme de coiffage du virus de la chlorelle, ou une enzyme de coiffage de S. *cerevisiae.*

25. Préparation ou trousse comprenant une enzyme de coiffage et un composé représenté par la Formule (I) : 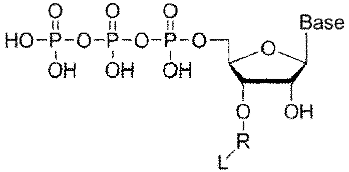 ou un sel de celui-ci, dans lequel :
la Base est une purine ou une pyrimidine,
R est un segment de liaison, dans lequel le segment de liaison est un groupe alkylène à chaîne linéaire ou ramifiée avec 1 à 300 atomes de carbone, et
L est un marqueur sélectionné parmi le groupe constitué par un marqueur d'affinité, un marqueur de détection, un groupe chimiosélectif, un oligonucléotide, et une combinaison de ceux-ci.

26. Préparation ou trousse selon la revendication 25, dans laquelle :
le marqueur de détection est un marqueur fluorescent ; ou dans laquelle le marqueur est un marqueur d'affinité sélectionné parmi le groupe constitué par un groupement biotine, la desthiobiotine, l'avidine, la streptavidine, une protéine A, une protéine de liaison au maltose, la poly-histidine, l'étiquette HA, l'étiquette c-myc, l'étiquette FLAG, l'étiquette SNAP, l'étiquette S et la glutathione-S-transférase (GST) ; préférablement un groupement biotine ou la desthiobiotine ; et/ou la base est une purine ; dans laquelle la purine est sélectionnée parmi une guanosine, une inosine ou un analogue de celle-ci.

27. Préparation ou trousse selon la revendication 26, dans laquelle le composé est le 3'-O-(2-aminoéthylcarbamoyl) (EDA)-biotin guanosine triphosphate (GTP) ou le 3'-desthiobiotin-tétraéthylène glycol (TEG)-GTP.

28. Préparation ou trousse selon l'une quelconque des revendications 25 à 27, dans laquelle l'enzyme de coiffage est une enzyme de coiffage de la vaccine (VCE), une enzyme de coiffage du virus de la fièvre catarrhale, une enzyme de coiffage du virus de la chlorelle, ou une enzyme de coiffage de S. *cerevisiae.*

29. Préparation ou trousse selon l'une quelconque des revendications 25 à 28, dans laquelle la trousse comprend en outre une matrice d'affinité.
